# EUROPÄISCHE PATENTSCHRIFT

(11) **EP 3 074 407 B1**
(45) Veröffentlichungstag und Bekanntmachung des Hinweises auf die Patenterteilung: **05.06.2019**
(21) Anmeldenummer: 14809780.1
(22) Anmeldetag: 26.11.2014
(51) Int. Cl.: A61K 51/04, A61K 51/08, C07B 59/00, C07F 13/00, C07F 9/50

(54) **VERBINDUNG UND VERFAHREN ZUR SELEKTIVEN RADIOMARKIERUNG VON POLYPEPTIDEN MITTELS FESTPHASENSYNTHESE**
COMPOUND AND METHOD FOR SELECTIVE RADIOLABELLING OF POLYPEPTIDES BY MEANS OF SOLID-PHASE SYNTHESIS
LIAISON ET PROCÉDÉ DE RADIOMARQUAGE SÉLECTIF DE POLYPEPTIDES PAR SYNTHÈSE EN PHASE SOLIDE

(30) Priorität: 28.11.2013 DE 102013113156
(43) Veröffentlichungstag der Anmeldung: 05.10.2016
(73) Patentinhaber: Freie Universität Berlin, 14195 Berlin (DE)
(72) Erfinder: BARANDOV, Ali, 14195 Berlin (DE); KOKSCH, Beate, 14195 Berlin (DE); ABRAM, Ulrich, 14195 Berlin (DE); ARAGHI, Raheleh Rezaei, 14195 Berlin (DE)
(74) Vertreter: Schiweck Weinzierl Koch Patentanwälte Partnerschaft mbB
(86) Internationale Anmeldenummer: PCT/EP2014/075594
(87) Internationale Veröffentlichungsnummer: WO 2015/078880

(56) Entgegenhaltungen:
- EP-B1- 0 644 778
- WO-A1-2010/066010
- WO-A2-01/68565

## Beschreibung

Die vorliegende Erfindung betrifft eine Verbindung und Verfahren zur selektiven Radiomarkierung von Peptiden und Polypeptiden mittels Festphasensynthese. Die Erfindung betrifft ferner die Verwendung von mittels dieses Verfahrens hergestellter markierten Peptide und Polypeptide und deren Verwendung zur Herstellung von bildgebenden Reagenzien und Strahlungstherapeutika. Die Erfindung betrifft weiterhin ein Kit zur Herstellung eines Radiopharmazeutikums oder einer radiodiagnostischen Zusammensetzung.

Mit Radioisotopen markierte Peptide werden in der Nuklearmedizin standardmäßig zur Therapie oder zur Lokalisation/Diagnostik bestimmter pathologischer Zustände mittels bildgebender Verfahren wie Gamma-Szintigraphie oder Einzelphotonen-Emissionscomputertomographie (SPECT, Single Photon Emission Computed Tomography) verwendet. Dabei werden diese markierten Peptide als Radiopharmaka oder Radiotracer verabreicht.

Beispiele für solche Radiopharmaka oder bildgebende Reagenzien sind zum Beispiel die in der Europäischen Patentschrift EP 0 762 901 B1 oder der US Patentschrift 6,667,389 B1 genannten Polypeptide. Diese Reagenzien bestehen aus einem spezifisch bindenden Peptid mit einer Aminosäuresequenz von 3 bis 100 Aminosäuren und einer damit kovalent verbundenen ^{99m}Technetium-bindenden Einheit. Dabei ist die ^{99m}Technetium-bindende Einheit kovalent an die Seitenkette eines Aminosäurerests des spezifisch bindenden Peptids gebunden, und weist geschützte Cysteine, die für die Komplexierung des Technetiums verantwortlich sind, auf. Weitere Beispiele von (mit Radionukliden) markierten Peptiden sind z.B. in den US Patentschriften US 6,017,510 US 5,443,815 beschrieben. Diese radiomarkierten Peptide haben üblicherweise eine kovalent gebundene, zur Metall-Komplexierung befähigte Einheit (d.h. eine Chelatorgruppe mit einem oder mehreren Mono- oder Multidentatliganden), mittels derer das gewünschte Radionuklid an das Peptid durch Komplexbildung gebunden wird. Der Herstellung solcher markierten Peptide ist gemeinsam, dass zunächst das gewünschte Peptid samt der zur Metall-Komplexierung befähigten Einheit mittels Festphasenpeptidsynthese hergestellt wird, das Peptid danach von der Festphase abgespalten, gereinigt und in Lösung mit dem Radionuklid komplexiert wird. Bei dieser Radiomarkierung können sich jedoch Probleme einstellen, z.B. können freie Cystein- oder Histidin-Seitenketten des Peptids mit der Chelatorgruppe bei der Bindung des Metallions konkurrieren und somit die Ausbeute an radioaktiv markiertem Peptid verringern. Ferner beschreibt die Internationale Patentanmeldung WO 01/68565 die Verwendung von Phosphin-Derivaten zur Modifikation rekombinanter Proteine, während die Internationale Patentanmeldung WO 2010/066010 die Herstellung von asymmetrischen Bi(thiosemicarbazonen), die beispielsweise bei bildgebenden Verfahren eingesetzt werden können.

Aufgabe der vorliegenden Erfindung ist es daher, ein Verfahren zur selektiven Markierung eines Peptids mit einem Radionuklid bereitzustellen, das reproduzierbar und einfach durchzuführen ist und von der Aminosäuresequenz des jeweiligen zu markierenden Peptides unabhängig ist.

Diese Aufgabe wird gelöst durch eine Verbindung sowie ein Verfahren mit den Merkmalen des jeweiligen unabhängigen Anspruchs.

Die Erfindung stellt daher in einer Ausführungsform eine Verbindung der Formel

T-Pep-BG (I)

bereit, wobei in Formel (I) T ein fester Träger ist;
Pep ein kovalent an T gebundenes Polypeptid ist, wobei das Polypeptid eine Aminosäuresequenz von mindestens 2 Aminosäuren, vorzugsweise eine Aminosäuresequenz mit 3 bis 200 Aminosäuren aufweist
BG eine (nicht-komplexierte) Bindungsgruppe für ein Metall M ist, das aus der Gruppe ausgewählt wird, die aus ^{99m}Tc, ¹⁸⁶Re, ¹⁸⁸Re, ⁹⁰Y, ⁸⁶Y,¹⁷⁷Lu, ⁶⁸Ga, ⁶⁷Cu, ⁶⁴Cu, ⁶⁷Ga, ⁸⁹Zr, ¹⁵³Sm und ¹¹¹In besteht,
wobei BG ausgewählt wird aus gegebenenfalls substituierten Aryl-Phosphanderivaten oder gegebenenfalls substituierten Heteroaryl-Phosphanderivaten der Formel (IIa)

P(R⁵)₃ (IIa),

wobei R⁵ gegebenenfalls substituiertes Aryl oder gegebenenfalls substituiertes Heteroaryl ist, wobei der Substituent von Aryl oder Heteroaryl ausgewählt wird aus R⁴-C₁-C₆ Alkyl, R⁴⁻NH-(C₁-C₆)Alkyl,R⁴-N=C-, R⁴-(C₁-C₆)Alkyl-NH-NH-(C₁-C₆)Alkyl, wobei R⁴ ausgewählt wird aus Aryl, Heteroaryl oder Heterocyclyl, die gegebenenfalls alle mit einem oder zwei Substituenten R⁶ substituiert sind, wobei R⁶ aus OH-(C₁-C₆) Alkyl, OH, NH₂, und (C₁-C₆)Alkoxy ausgewählt wird,
und wobei zumindest einer der Reste R⁵ mit einem Substituenten R¹ versehen ist, der aus -CO-, -S-, -NH- oder -O- ausgewählt wird, und
wobei die Gruppe BG mittels des Substituenten R¹ mit einer reaktiven Seitenkette einer Aminosäure von Pep oder der Alpha-Aminogruppe oder der Carboxylgruppe einer Aminosäure von Pep kovalent verbunden ist.

In einer weiteren Ausführungsform stellt die Erfindung ein Verfahren bereit zur Herstellung einer Verbindung der Formel (I)

T-Pep-BG (I)

wobei T in Formel (I) T ein fester Träger ist;
wobei das Verfahren umfasst:
   a) Synthetisieren eines Polypeptids Pep, wobei Pep ein Polypeptid ist, das kovalent an den Träger T gebunden wird und eine Aminosäuresequenz mit mindestens 2 Aminosäuren, vorzugsweise eine Aminosäuresequenz mit 3 bis 200 Aminosäuren aufweist,
      um eine Verbindung der Formel

      T-Pep

      zu erhalten;
   b) Umsetzen von T-Pep mit einem Vorläufermolekül BG' von BG, um eine Verbindung der Formel (I)

      T-Pep-BG (I)

      zu erhalten, wobei BG eine Bindungsgruppe für ein Metall M ist, wobei das Metall M aus der Gruppe ausgewählt wird, die ^{99m}Tc, ¹¹⁶Re, ¹⁸⁸ Re, ⁹⁰Y, ⁸⁶Y, ¹⁷⁷Lu, ⁶⁸Ga, ⁶⁷Cu, ⁶⁴Cu, ⁶⁷Ga, ⁸⁹Zr, ¹⁵³Sm und ¹¹¹In besteht.

Die vorliegende Erfindung beruht, anschaulich ausgedrückt, auf der überraschenden Erkenntnis, dass bei der Festphasensynthese das herzustellende Polypeptid zunächst kovalent mit einer Bindungsgruppe BG (z.B. einer Chelatorgruppe) verknüpft werden kann, und diese, noch an den Träger gebundene Verbindung T-Pep-BG (I) zur Komplexierung/Radiomarkierung eingesetzt werden kann. Dies hat den Vorteil, dass die hier betrachteten radioaktiven Metalle wie ^{99m}Tc, die kommerziell als einfache wässrige Lösung erhältlich sind, in dieser Form direkt zur Markierung des Polypeptids verwendet werden können. Dabei kann, falls gewünscht, zu einer wässrigen Lösung des radioaktiven Metalls auch ein organisches Lösungsmittel wie z.B. Dimethylformamid (DMF), Dimethylsulfoxid (DMSO) oder Ethanol hinzugegeben werden, um so den Aufnahmegrad der Radioaktivität durch die Verbindung T-Pep-BG (I) noch weiter zu verbessern. Von Vorteil ist in diesem Zusammenhang ebenfalls, dass es durch das Verfahren der Erfindung möglich ist, nur sehr geringe Konzentration des radioaktiven Metalls (die im nano- bis zum picomolaren Bereich liegen können) zur Markierung einzusetzen. Das Verfahren der Erfindung hat zudem den Vorteil, dass es leicht standardisiert werden kann, da die Festphasensynthese für jedes gewünschte Polypeptid unter den prinzipiell selben Bedingungen erfolgen kann. Somit eröffnet die Erfindung den Weg zu einem einfachen, kostengünstigen und zugleich automatisierbaren Verfahren zur Markierung eines Polypeptids mit einem radioaktiven Metall.

Im Einklang mit der vorstehenden Offenbarung wird beim Verfahren der vorliegenden Erfindung nach der Markierung das so markierte Polypeptid Pep vom festen Trägermaterial abgespalten. Das Verfahren umfasst daher in einer Ausführungsform:
c) Umsetzen der Verbindung der Formel (I) mit einem Reagenz, das ein Metall M umfasst, um eine Verbindung der Formeln (IIa), (IIb) oder (IIc)

   T-Pep-BG-M(Y1)(Y2)(Y3) (IIa)

   T-Pep-BG-M(Y1)(Y2) (IIb)

   oder

   T-Pep-BG-M(Y1) (IIc)

   zu bilden, wobei
   Y1, Y2 und Y3 jeweils unabhängig voneinander CO oder Cl sind, oder eines von Y1, Y2 oder Y3 O ist und die beiden anderen unabhängig voneinander CO oder Cl sind, und
   M aus ^{99m}Tc, ¹⁸⁶Re, ¹⁸⁸Re, ⁹⁰Y, ⁸⁶Y, ¹⁷⁷Lu, ⁶⁸Ga, ⁶⁷Cu, ⁶⁴Cu, ⁶⁷Ga, ⁸⁹Zr, ¹⁵³Sm und ¹¹¹In ausgewählt wird; und
d) gegebenenfalls Spalten der Bindung zwischen T und Pep, um eine Verbindung der Formeln (IIIa), (IIIb) or (IIIc)
H-Pep-BG-M(Y1)(Y2)(Y3) (IIIa)

,

H-Pep-BG-M(Y1)(Y2) (IIIb)

oder

H-Pep-BG-M(Y1) (IIIc)

zu erhalten.

Im Nachfolgenden wird die Erfindung detailliert beschrieben.

Das zu markierende Polypeptid Pep kann jedes beliebige Polypeptid sein, das mehr als 2 Aminosäurereste besitzt/umfasst. Das Polypeptid kann daher beliebig groß sein und eine Größe von bis zu mehreren hundert Aminosäuren besitzen. Üblicherweise besitzt das Polypeptid Pep bis zu 300, 250 oder 200 Aminosäurereste. Das Polypeptid im Sinne der Erfindung umfasst daher auch Tripeptide und Oligopeptide (die üblicherweise eine Länge von bis zu zehn Aminosäuren aufweisen). In Ausführungsformen der Erfindung kann ein Polypeptid Pep verwendet werden, das 3 bis 300, 3 bis 250, 3 bis 200, 3 bis 150, 3 bis 120, 3 bis 100, 3 bis 50, 3 bis 40, 3 bis 40 oder 3 bis 25, 3 bis 22, 3 bis 20, 3 bis 17, 3 bis 15, oder 3 bis 10 Aminosäuren aufweist. Das Polypeptid kann reaktive Seitenketten mit -SH oder OH- oder -NH₂-Grupen aufweisen, die ggf. bei der Markierung eines Peptids durch ihre Affinität für Metallionen mit der Bindung der Bindungsgruppe BG mit dem Metall M interferieren können. Aus diesem Grund können solche reaktiven Seitenketten in der Verbindung nach Formel (I) durch eine der dem Fachmann auf dem Gebiet der Peptidsynthese bekannten Schutzgruppen geschützt sein.

Das Polypeptid kann einen speziellen Bindungspartner aufweisen, mit dem es eine physiologisch relevante Interaktion eingehen kann, die z.B. zur Diagnostik oder Therapie verwendet werden kann. Das Polypeptid kann sowohl ein lineares Peptid als auch ein cyclisches Peptid sein.

Als erstes rein illustratives Beispiel sei hier das Neuropeptid Substanz P (SP) diskutiert. Substanz P (SP) ist ein 11 Aminosäuren langes Neuropeptid mit der Sequenz Arg Pro Lys Pro Gln Gln Gly Phe Phe Leu Met (SEQ ID NO: 1), das als Neurotransmitter und als Neuromodulator fungiert und zur Tachykinin Neuropeptid Familie gehört. Substanz P und dessen eng verwandtes Neuropeptid Neurokinin A (NKA) werden aus einem Vorläufer-Polyprotein, dem Preprotachykinin, durch differentielles Spleißen des Gens hergestellt. Substanz P wurde zunächst als Neurotransmitter bei Schmerzrezeptoren (Nozizeptor) und schmerzleitenden C-Fasern angesehen. Substanz P bewirkt eine starke Erweiterung der Blutgefäße und steigert die Durchlässigkeit der Gefäßwand. Zudem bewirkt Substanz P eine Steigerung der Sensitivität der Schmerzneurone im Rückenmark. Substanz P reguliert auch die zielgerichtete Einwanderung von Leukozyten (Chemotaxis). Leukozyten exprimieren sowohl Substanz P als auch den Substanz P-Rezeptor (Neurokinin-1 Rezeptor, NK-1R).

Diagnostisch wird Substanz P z.B. zur Darstellung des Thymus in Patienten mit immun-vermittelten Krankheiten eingesetzt (siehe z.B. Van Hagen et al. Visualization of the thymus by substance P receptor scintigraphy in man. Eur J Nucl Med. 1996 Nov; 23(11):1508-13).

Ein weiteres illustratives Beispiel sind Peptide wie GNGRG (SEQ ID NO: 2) oder (GNGRG)₂KNK (SEQ ID NO: 3), die auf dem Tripeptid Asn-Gly-Arg (NGR) basieren und die die Aminopeptidase N (APN oder CD13) auf der Membran von Tumorzellen erkennen, und dadurch in der Lage sind, sich spezifisch auf Tumorgefäßsysteme zu richten, siehe hierzu den Übersichtsartikel von Wang et al. "Development of NGR peptide-based agents for tumor imaging" Am J Nucl Med Mol Imaging 2011;1(1):36-46. Solche erfindungsgemäß markierten Peptide können Einsatz in der Tumortherapie oder Tumordiagnostik finden. Ein anderes Beispiel sind Peptide auf Basis des Tripeptids Arg-Gly-Asp (RGD), wie sie z.B. von Varshney et al. "Solid phase synthesis, radiolabeling and biological evaluation of a 99mTclabeled αVβ3 tripeptide (RGD) conjugated to DOTA as a tumor imaging agent", Vol. 11, 10. Mai 15, 2011, Seiten 893 - 901 beschrieben sind.

Weitere anschauliche Beispiele von Peptiden, die in der Erfindung verwendet werden können, beinhalten die folgenden Peptide und Fragmente davon:
- Formyl MLF (der Prototyp von Formylpeptiden, die Neutrophilrezeptoren binden)
- (VGVAPG)₃ (SEQ ID NO: 4)
- VPGVG (SEQ ID NO: 5)
- RALVDTLKFVTQAEGAK (SEQ ID NO: 6)
- RALVDTEFKVKQEAGAK (SEQ ID NO: 7)
- PLARITLPDFRLPEIAIP (SEQ ID NO: 8)
- GQQHHLGGAKAGDV (SEQ ID NO: 9)
- PLYKKIIKKLLES (SEQ ID NO: 10)
- LRALVDTLK (SEQ ID NO: 11)
- GGGLRALVDTLK (SEQ ID NO: 12)
- GGGLRALVDTLKFVTQAEGAK (SEQ ID NO: 13)
- GGGRALVDTLKALVDTL (SEQ ID NO: 14)
- GHRPLDKKREEAPSLRPAPPPISGGGYR (SEQ ID NO: 15)
- PSPSPIHPAHHKRDRRQ (SEQ ID NO: 16)
- GGGFD (SEQ ID NO: 17)
- YWDKTFT SEQ ID NO: 18)
- SYNRGDSTC (SEQ ID NO: 19)
- GGGLRALVDTLK (SEQ ID NO: 20)
- GCGGGLRALVDTLK (SEQ ID NO: 21)
- GCYRALVDTLKFVTQAEGA (SEQ ID NO: 22), oder
- GC(VGVAPG)₃ (SEQ ID NO: 23)
- Neuropeptid Y (36 Aminosäuren Länge (AS) und funktionelle Fragmente davon)
- Somastin (92 AS Länge und funktionelle Fragmente davon)
- Cholecystokinin (95 AS Länge und funktionelle Fragmente davon)
- Pankrezymin (95AS Länge und funktionelle Fragmente davon)
- Bombesin (14 AS Länge und funktionelle Fragmente davon)
- Galanin (30 AS Länge und funktionelle Fragmente davon)
- Bradykinin (9 AS Länge und funktionelle Fragmente davon)
- Angiotensin II (8 AS Länge und funktionelle Fragmente davon) und
- Calcitonin Gen-Related Peptide (CGRP) (SEQ ID NO: 24) (37 AS Länge und funktionelle Fragmente davon).

In diesem Zusammenhang sei erwähnt, dass das Peptid Pep sowohl N-als auch C-terminal Aminosäuren aufweisen kann, die in der natürlichen Wildtypsequenz des Peptids P nicht vorkommen. Solche z.B. als "Linker" oder Abstandshalter (spacer) dienende Aminosäuren können z.B. bei einer vorherigen rekombinanten Herstellung (Synthese) des Peptids in dessen Aminosäuresequenz eingeführt werden. Hierbei sei auch erwähnt, dass es möglich ist, nicht das gesamte Peptid z.B. durch chemische Festphasensynthese herzustellen, insbesondere bei längeren Peptiden mit mehr als 100 Aminosäuren kann dies schwierig sein. In diesen Fällen kann jedoch zumindest ein Teil des gewünschten Peptids zunächst rekombinant (gentechnisch) hergestellt werden und dieses Peptid dann zur Markierungsreaktion in einer Festphasensynthese eingesetzt werden. N- und C-terminale Aminosäuren, die nicht in der natürlichen Aminosäuresequenz von Pep vorkommen, können ebenfalls im Rahmen der Festphasensynthese eingeführt werden. Im Rahmen der Synthese, sowohl der Festphasensynthese oder aber nach einer (zumindest teilweisen) Herstellung des Peptids Pep können auch Linker, die nicht auf Aminosäuren beruhen, in das Peptid eingefügt werden. Gleichfalls ist es möglich, dass das Polypeptid Pep auch noch andere Modifikationen aufweist, z.B. kann das Peptid nach der Synthese mit halbwertszeitverlängernde Polyoxyalkylenmolekülen wie Polyethylenglykole verschiedener Molekulargewichte konjugiert werden. Auch ist es möglich, das Polypeptid, sowohl während oder auch nach der Synthese mit z.B. halbwertszeitverlängernden Peptidsequenzen, die aus den Aminosäuren Pro, Ala, bestehen, zu fusionieren oder zu konjugieren. Diese auch als "PASylation®" bekannte Technologie ist z.B. in den Europäischen Patentschriften 2 173 890 B2 und EP 2 369 005 beschrieben. Abschließend sei angemerkt, dass das hier verwendete Polypeptid Pep selbstverständlich nicht auf natürlich vorkommende Polypeptide beschränkt ist, sondern auch Mutationen in der Aminosäuresequenz im Vergleich zu einer natürlich vorkommenden Sequenz aufweisen kann. Das Polypeptid kann weiterhin auch eine vollständig künstliche Aminosäuresequenz aufweisen.

Die im Kontext der beanspruchten Erfindung offenbarte Bindungsgruppe BG für das Metall M kann jede beliebige Gruppe sein, die in der Lage ist, koordinativ an das Metall M (^{99m}Tc, ¹⁸⁶Re, ¹⁸⁸Re, ⁹⁰Y, ⁸⁶Y,¹⁷⁷Lu, ⁶⁸Ga, ⁶⁷Cu, ⁶⁴Cu, ⁶⁷Ga, ⁸⁹Zr, ¹⁵³Sm und ¹¹¹In) zu binden und gleichzeitig kovalent mit dem Polypeptid Pep verknüpft ist. Beispiele solcher Gruppen sind Chelatoren, die Mono- oder Multidentat-Liganden aufweisen und die aus dem Stand der Technik wohl bekannt sind. So können z.B. für die hier betrachteten Metalle als Bindungsgruppe BG in Verbindungen der Formel (I) die polyazamakrocyclischen Chelatoren, die in US Patent 8,198,101 beschrieben sind, verwendet werden. Diese Chelatoren besitzen die allgemeine Formel (für die genaue Definition der Formel sei auf die US-Patentschrift 8,198,101 verwiesen) 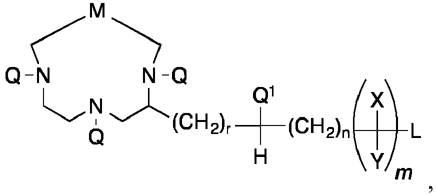 die in der Linkergruppe L eine reaktive Gruppe trägt, mit der die Chelatoren kovalent mit dem Polypeptid Pep verknüpft werden können. Weitere für die Komplexierung der hier betrachteten Metalle geeignete Chelatoren sind in der US Patentanmeldung US 2011/0313130 offenbart. Auch diese Chelatoren der allgemeinen Formel (für die genaue Definition der Formel sei auf die US-Veröffentlichungsschrift US 2011/0313130 verwiesen) 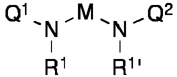 besitzen eine reaktive Gruppe, mit der die Chelatoren kovalent mit dem Polypeptid Pep verknüpft werden können.

Ein weiteres Beispiel für Chelatoren, die als im Kontext der beanspruchten Erfindung offenbarte Bindungsgruppe BG in Verbindungen der Formel (I) verwendet werden können, sind Pyrazolyl-enthaltende mehrzähnige Chelatoren der folgenden allgemeinen Formeln, die in Correia et al. Current Radiopharmaceuticals Vol. 2, Nr. 4, Oktober 2009 Seiten 277-294, beschrieben sind, und bei denen die Konjugationsstellen für die kovalente Verknüpfung mit dem Polypeptid Pep mit einem Pfeil gekennzeichnet sind:

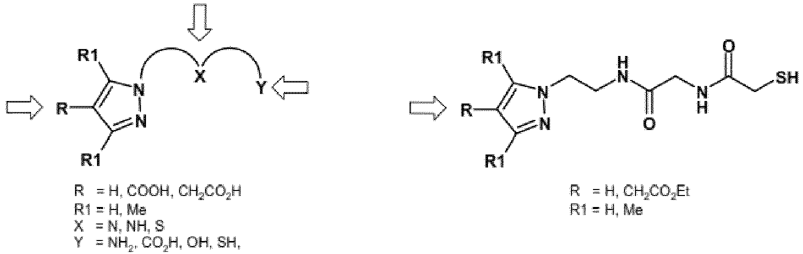

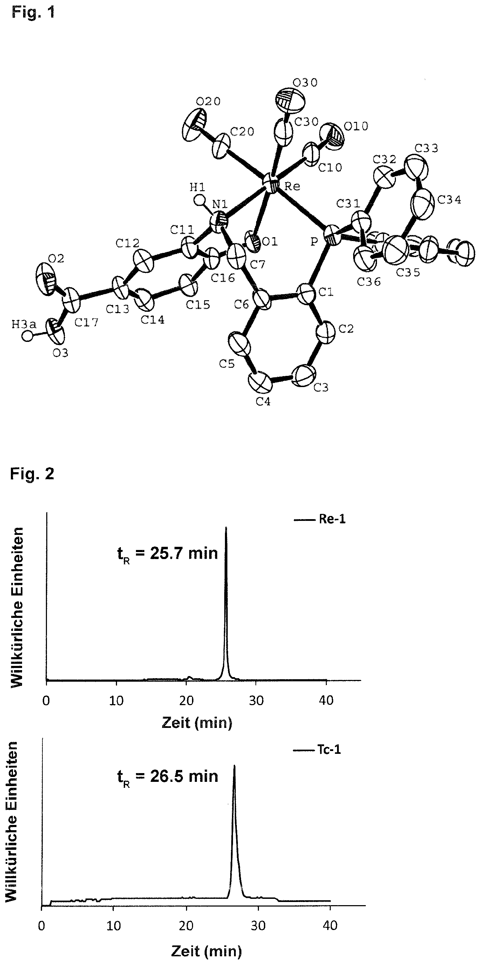

Weitere illustrative Beispiele solcher im Kontext der beanspruchten Erfindung offenbarten Liganden schließen zum Beispiel Chelatoren für Radionuklide ein, die z.B. mit Polypeptiden wie Antikörpern oder deren Antigen bindenden Fragmente kovalent verknüpft werden können, um so radiomarkierte Antikörpermoleküle zu erhalten. Beispiele solcher Chelatoren sind z.B. die pentavalenten Dimercaptobernsteinsäure-Derivate (DMSA von engl. Dimercaptosuccinic acid), die in Fig. 16.7, Seite 330 TECHNETIUM-99m RADIOPHARMACEUTICALS: STATUS AND TRENDS. Vienna: International Atomic Energy Agency, 2009*. (*IAEA radioisotopes and radiopharmaceuticals series, ISSN 2077-6462; no. 1) STI/PUB/1405 ISBN 978-92-0-103509-7 beschrieben sind, die in Fig. 14.2, Seite 286 dieser Publikation beschriebenen, an Oligopeptide konjugierten Bisphosphonate oder die Biokonjugate mit den im Abschnitt 2.3 dieser Publikation beschriebenen Ligandensystemen auf Basis von z.B. vierzähnigen reinen Stickstoff (N-) oder Stickstoff/Schwefel (NS-), oder Stickstoff/Sauerstoff (NO)-Liganden. Illustrative Beispiele solcher Liganden sind die in Fig. 2.1. von TECHNETIUM-99m RADIOPHARMACEUTICALS: STATUS AND TRENDS, supra, und hier ebenfalls nachfolgend abgebildeten Liganden.

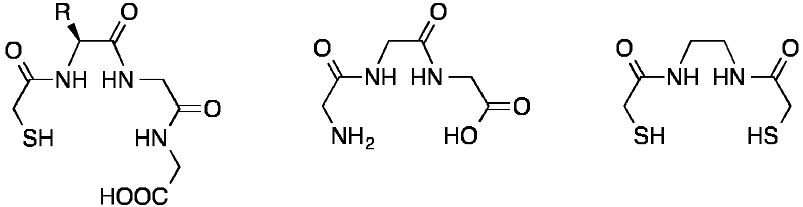

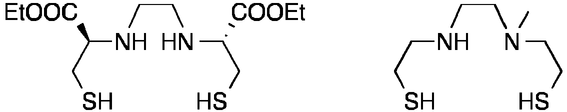

Weitere illustrative Beispiele für als Gruppe BG geeignete Chelatoren sind die heterofunktionalen in den beanspruchten Verbindungen verwandten Phosphan-Liganden, die im nachfolgenden genauer beschrieben werden.

Die Bindungsgruppe BG kann an einer beliebigen Stelle des Peptids Pep mit diesem kovalent verbunden sein. In einigen Ausführungsformen der Verbindung der Formel (I) ist die Bindungsgruppe BG an die alpha-Aminogruppe des N-terminalen Aminosäurerests von Pep, an eine Carboxylgruppe einer Aminosäure von Pep oder an eine Seitenkette eines innerhalb von Pep angeordneten Aminosäurerests gebunden. Die kovalente Verknüpfung der Bindungsgruppe BG mit dem Polypeptid Pep kann auf bekannte Weise erfolgen, beispielsweise wie in Kapitel 2.1 auf Seite 24 der Publikation TECHNETIUM-99m RADIOPHARMACEUTICALS: STATUS AND TRENDS, supra, anhand der Markierung eines Fragments von Bombesin beschrieben. Wie oben erwähnt, kann das Peptid Pep reaktive Seitenkettengruppen wie -OH, -NH₂ oder -SH aufweisen. Daher können reaktive Seitenketten in einer Verbindung der Formel (I) durch dem Fachmann bekannte Schutzgruppen geschützt sein. Dem Fachmann bekannte Schutzgruppen für Seitenketten von Peptiden und Polypeptiden schließen *tert*-Butoxycarbonyl (Boc), Fluorenylmethoxycarbonyl (Fmoc) (üblicherweise verwendet zum Schutz der OH-Gruppe in Ser oder Thr), die 2,6-Dichlorbenzylschutzgruppe (üblicherweise verwendet zum Schutz der OH-Gruppe in Tyr), Benzylester (üblicherweise für die Carboxylgruppen von Asp und Glu verwendet), die 2-Chlorbenzyl-oxycarbonylschutzgruppe (2ClZ) oder die Benzyloxycarbonylschutzgruppe (üblicherweise zum Schutz der freien Aminogruppe von Lys), 4-Toluol-sulfonyl (Tos) und die Mesitylen-2-sulfonyl-Gruppe (Mts) (üblicherweise verwendet zum Schutz der Guanidiniumgruppe von Arg), Tosyl oder 2,4-Dinitrophenylschutzgruppe (DNP) zum Schutz des N^{τ} Atoms von His) ein, sind aber nicht auf diese beschränkt.

Im Einklang mit der vorstehenden Offenbarung wird in der beanspruchten Erfindung die Bindungsgruppe BG ausgewählt aus gegebenenfalls substituierten Aryl-Phosphanderivaten oder gegebenenfalls substituierten Heteroaryl-Phosphanderivaten der Formel

P(R⁵)₃ (IIa),

wobei in Formel (IIa) R⁵ gegebenenfalls substituiertes Aryl oder gegebenenfalls substituiertes Heteroaryl ist, wobei der Substituent von Aryl oder Heteroaryl ausgewählt wird aus R⁴-C₁-C₆ Alkyl, R⁴-NH-(C₁-C₆)Alkyl, R⁴-N=C-, R⁴-(C₁-C₆)Alkyl-NH-NH-(C₁-C₆)Alkyl, wobei R⁴ ausgewählt wird aus Aryl, Heteroaryl oder Heterocyclyl, die gegebenenfalls alle mit einem oder zwei Substituenten R⁶ substituiert sind, wobei R⁶ aus OH-(C₁-C₆) Alkyl, OH, NH₂, und (C₁-C₆)Alkoxy ausgewählt wird,
und wobei zumindest einer der Reste R⁵ mit einem Substituenten R¹ versehen ist, der aus - CO-, -S-, -NH- oder -O- ausgewählt wird.

Dabei ist in Verbindungen der Formel (I) die Gruppe BG oder das Phosphanderivat von Formel (IIa) mittels des Substituenten R¹ mit einer reaktiven Seitenkette einer Aminosäure von Pep, der Alpha-Aminogruppe von Pep (d.h. der freien N-terminalen Aminogruppe von Pep) oder der Carboxylgruppe einer Aminosäure von Pep kovalent verbunden.

Alkylreste, wenn hier in Formeln genannt, können geradkettig oder verzweigt sein. Dies gilt auch, wenn sie in anderen Gruppen enthalten sind, zum Beispiel in Alkoxygruppen, Alkoxycarbonylgruppen oder in Aminogruppen, oder wenn sie substituiert sind.

Beispiele für Alkylgruppen sind Methyl, Ethyl, Propyl, Butyl, Pentyl, Hexyl, oder die n-Isomeren dieser Reste, Isopropyl, Isobutyl, Isopentyl, secButyl, tert-Butyl, Neopentyl, 3,3-Dimethylbutyl. Unter dem Begriff "Alkyl" sind hier ausdrücklich auch ungesättigte Alkylreste zu verstehen, also Alkylreste, die eine oder mehrere Doppelbindungen und/oder eine oder mehrere Dreifachbindungen enthalten, also Alkenylreste und Alkinylreste. Beispiele für solche Reste sind der Vinylrest, der 2-Propenylrest (Allylrest), der 2-Butenylrest, der 2-Methyl-2-propenylrest, der Ethinylrest, der 2-Propinylrest (Propargylrest) oder der 3-Butinylrest. Weiterhin sind unter dem Begriff Alkyl hier ausdrücklich auch Reste zu verstehen, in denen durch einen internen Ringschluss ein cyclisches System gebildet wird, der Begriff Alkyl umfasst also auch gesättigte und teilweise ungesättigte Cycloalkylreste und Cycloalkylalkylreste (durch Cycloalkyl substituiertes Alkyl, wie z. B. Cyclohexylmethyl. Beispiele für solche Cycloalkylreste sind Cyclopropyl, Cyclobutyl, Cyclopentyl, Cyclohexyl, Cycloheptyl und Cycloctyl, die alle auch zum Beispiel durch einen oder mehrere gleiche oder verschiedene (C₁-C₄)-Alkylreste, insbesondere durch Methyl, substituiert sein können. Beispiele für solche substituierten Cycloalkylreste sind 4-Methylcyclohexyl, oder 2,3-Dimethylcyclopentyl.

Der hier verwendete Begriff "Aryl" bezieht sich auf einen carbozyklischen oder heterocyclischen aromatischen Rest, der einen, zwei oder drei Ringe enthält, wobei diese Ringe miteinander verbunden oder anniliert sein können. Der Begriff "Aryl" schließt daher z.B. Phenyl, Naphthyl, Indenyl, Tetrahydronaphthyl, Dihydrobenzofuranyl, Anthracenyl, Andanyl, und Benzodioxazinyl ein. Arylreste können unsubstituiert oder substituiert sein, üblicherweise mit einem bis fünf Substituenten. Substituenten können eine oder mehrere gleiche oder verschiedene organische Reste sein, zum Beispiel C₁-C₆ Alkyl, Hydroxyl, Halogen, Halo-C₁-C₆-Alkyl, Nitro, Cyano, C₁-C₆-Alkoxy, C₁-C₆ Halogen, -OH, oder -O-C₁-C₆-Alkyl.

Beispiele für heterocyclische Reste, von denen sich in den Verbindungen vorkommende Reste ableiten können, sind Pyrrol, Furan, Thiophen, Imidazol, Pyrazol, 1,2,3-Triazol, 1,2,4-Triazol, 1,3-Oxazol, 1,2-Oxazol, 1,3-Thiazol, 1,2-Thiazol, Tetrazol, Pyridin, Pyridazin, Pyrimidin, Pyrazin, Pyran, Thiopyran, 1,4-Dioxin, 1,2-Oxazin, 1,3-Oxazin, 1,4-Oxazin, 1,2-Thiazin, 1,3-Thiazin, 1,4-Thiazin, 1,2,3-Triazin, 1,2,4-Triazin, 1,3,5-Triazin, 1,2,4,5-Tetrazin, Azepin, 1,2-Diazepin, 1,3-Diazepin, 1,4-Diazepin, 1,3-Oxazepin oder 1,3-Thiazepin.

In einigen Ausführungsformen der Verbindung der Formel (I) ist BG eine Gruppe der Formel (A), (B), (C), (D), (E), (F) oder (G) : 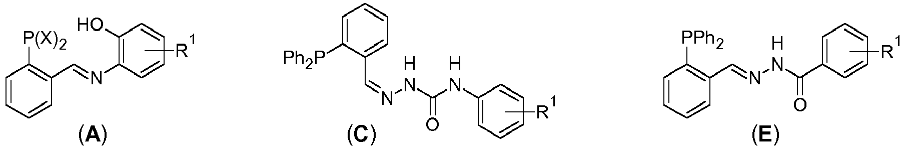 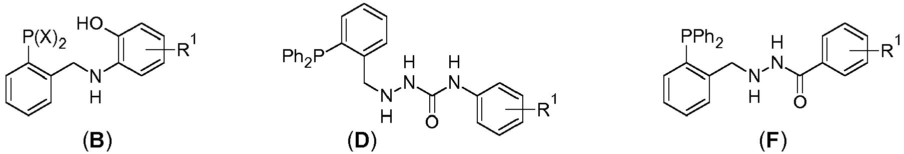 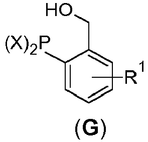
wobei in den Verbindungen der Formeln (A), (B), (C), (D), (E), (F), oder (G) die Gruppe BG mittels des Substituenten R¹ kovalent mit einer Seitenkette einer Aminosäure von Pep oder mit der Alpha-Aminogruppe von Pep oder der Carboxylgruppe einer Aminosäure von Pep verbunden ist,
wobei in den Formeln (A), (B), (C), (D), (E), (F), oder (G)
R¹ aus -CO-, -S-, -NH-, oder -O- ausgewählt ist;
X, jeweils unabhängig voneinander ausgewählt wird aus einer gegebenenfalls substituierten Arylgruppe, wobei die Substituenten ausgewählt werden aus (C₁-C₆)Alkyl, (C₁-C₆) Alkoxy, Hydroxy-(C₁-C₆) Alkyl, oder X eine Gruppe der Formeln (a) oder (b) 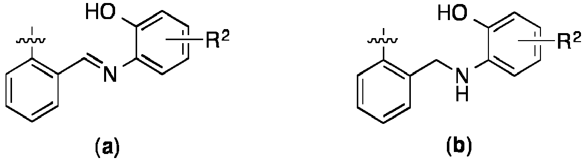 ist, wobei
   R² H, (C₁-C₆)Alkyl, (C₁-C₆)Alkoxy, Halogen, OH, NH₂, -CN, Aryl, SH, oder
   -S(C₁-C₆)-Alkyl ist.

Solche Phosphin-Liganden der oben genannten Formeln (A), (B), (C), (D), (E), (F) oder (G) sind an sich bekannt und z.B. in der Dissertation von A. Barandov "Complexes of Rhenium and Technetium with Multidentate Phosphin Ligands", FU Berlin, 2009 Fachbereich Biologie, Chemie, Pharmazie beschrieben.

In einigen Ausführungsformen der Verbindung der Formel (I) im allgemeinen oder der Formeln (A), (B), (C), (D), (E), (F) oder (G) ist der Rest R¹ in meta-Position zu der NH-Gruppe desselben Arylrings angeordnet. Dabei kann in einigen Ausführungsformen R¹ - CO sein. Der Rest X kann in solchen Verbindungen von Formel (I) Phenyl oder gegebenenfalls substituiertes Phenyl sein.

Die Bindungseinheit BG kann an jeder beliebigen Stelle des Polypeptids mit diesem kovalent verknüpft sein, beispielsweise mit der C-terminalen Aminosäure, der N-terminalen Aminosäure, aber, im Falle von reaktiven Seitenketten (-SH, -OH oder NH₂-Gruppen) auch an einer solchen Seitenkette innerhalb des Polypeptids.

Der feste Träger T kann jedes Material sein, das als Festphase bei der Festphasenpeptidsynthese eingesetzt werden kann. Solche (im Lösungsmittel für die Peptidsynthese unlöslichen) Träger sind auch als Harz bekannt. Beispiele für geeignete Trägermaterialien, die als fester Träger T in einem (z.B. auch automatisierten) Festphasenpeptidsynthese-Verfahren der Erfindung eingesetzt werden können, sind organische und anorganische Trägermaterialien wie Polystyrol-Harz nach Merrifield (Copolymer aus Styrol und 1-2% Divinylbenzol), Polyacrylamidharze, TentaGel (ein Pfropfpolymer von Polyethylenglycol, das an Polystyrol verankert ist), Wang-Harz (üblicherweise quervernetzes Polystyrol wie bei Merrifield-Harz als Grundgerüst) oder poröses Glas mit definierter Porengröße als Beispiel eines anorganischen Trägermaterials. Illustrative Beispiele für kommerziell erhältliche Trägermaterialien sind Rink Amid-Linkerharze oder NovaSyn®TGR-Harze, die von Merck Millipore bezogen werden können.

Wie eingangs bereits erwähnt, umfasst das erfindungsgemäße Verfahren, den Schritt c) die den Träger T umfassende Verbindung der Formel der Verbindung der Formel (I) mit einem Reagenz, das ein Metall M umfasst, umzusetzen, um eine Verbindung der Formel (IIa), (IIb) oder (IIc)

T-Pep-BG-M(Y1)(Y2)(Y3) (IIa)

,

T-Pep-BG-M(Y1)(Y2) (IIb)

oder

T-Pep-BG-M(Y1) (IIc)

zu bilden,
wobei
Y1, Y2 und Y3 jeweils unabhängig voneinander CO oder Cl sind, oder eines von Y1, Y2 oder Y3 O ist und die beiden anderen unabhängig voneinander CO oder Cl sind, und
M aus der Gruppe ausgewählt wird, die aus ^{99m}Tc, ¹⁸⁶Re, ¹⁸⁸Re, ⁹⁰Y, ⁸⁶Y,¹⁷⁷Lu, ⁶⁸Ga, ⁶⁷Cu, ⁶⁴Cu, ⁶⁷Ga, ⁸⁹Zr, ¹⁵³Sm und ¹¹¹In besteht; und
gegebenenfalls den Schritt d) Spalten der Bindung zwischen T und Pep, um eine Verbindung der Formel (IIIa), (IIIb) oder (IIIc)

H-Pep-BG-M(Y1)(Y2)(Y3) (IIIa)

,

H-Pep-BG-M(Y1)(Y2) (IIIb)

oder

H-Pep-BG-M(Y1) (IIIc)

zu erhalten.

Falls in dem Verfahren eine Verbindung der Formel (I) eingesetzt wurde, bei der eine oder mehrere (reaktiven) Seitenketten von Pep geschützt vorliegen, umfasst das Verfahren vor dem Schritt d) gegebenenfalls das Entschützen der geschützten Seitenketten.

Bei einigen Ausführungsformen dieses Verfahrens nach Schritt c) sind in dem Reagenz, das das Metall M umfasst, Y1, Y2 und Y3 jeweils CO. In einigen Ausführungsformen des Verfahrens, bei denen Y1, Y2 und Y3 jeweils unabhängig voneinander CO oder Cl sind, oder eines von Y1, Y2 oder Y3 O ist, und die beiden anderen Substituenten (z.B. Y2 und Y3) unabhängig voneinander CO oder Cl sind, ist das Metall M ^{99m}TC, ¹⁸⁶ Re oder ¹⁸⁸ Re.

Das Vorläufermolekül BG', das die Bindungsgruppe BG in der Verbindung nach Formel (I) oder (II) für die spätere Metallkomplexierung beisteuert, kann eine Verbindung der Formel (VI) 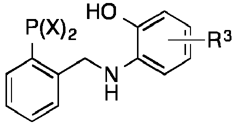 sein, wobei in der Verbindung von Formel (VI)
der Substituent R³ aus COOH, SH, NH₂, OH oder Halogen ausgewählt werden kann und
X jeweils unabhängig voneinander ausgewählt wird aus einer gegebenenfalls substituierten Arylgruppe, wobei die Substituenten ausgewählt werden aus (C₁-C₆) Alkyl, (C₁-C₆) Alkoxy, Hydroxy- (C₁-C₆) Alkyl, oder X eine Gruppe der Formeln (a) oder (b) 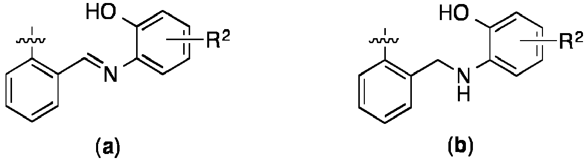 ist, wobei in den Formeln (a) und (b) R² H, (C₁-C₆) Alkyl, (C₁-C₆) Alkoxy, Halogen, OH, NH₂, -CN, Aryl, SH, oder -S(C₁-C₆)-Alkyl ist.

In diesem Verfahren kann R³ in der Verbindung von Formel (VI) in meta-Position zur NH Gruppe desselben Phenylrings angeordnet sein.

Die Gruppe M(Y1)(Y2)(Y3) kann in den Verbindungen der Formeln (II) und (III) einen Komplex der folgenden Struktur (bb) bilden, wenn zur Metallkomplexierung eine Gruppe der Formel (VI) verwendet wird: 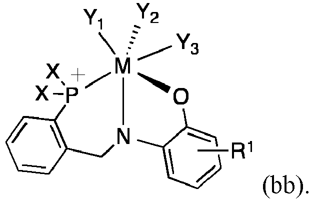
Dabei kann in diesem Komplex jeder Ligand Y1, Y2 und Y3 unabhängig voneinander aus CO und Cl ausgewählt werden, oder eines der Liganden Y1, Y2 oder Y3 stellt O dar und die beiden anderen Liganden stellen unabhängig voneinander CO oder Cl dar, und
jedes X ist Aryl, vorzugsweise Phenyl, und
der Substituent R1 kann aus -CO-, -S-, -NH- oder -O- ausgewählt werden. Diese Metallkomplexierung kann unter bekannten Bedingungen für die Komplexbildung erzielt werden und hängt von dem eingesetzten Metall ab. Die Komplexbildung mit dem Metall M erfolgt üblicherweise im wässriger Lösung oder einem Gemisch von Wasser mit einem organischen Lösungsmittel wie Ethanol, Ethylenglycol, Propylenglycol, DMSO oder DMF bei Raumtemperatur oder erhöhter Temperatur. Zum Beispiel kann im Einklang mit der oben angegeben Offenbarung die Komplexierung mit ^{99m}Tc, oder Re unter Verwendung eines Reagenzes wie die Metallcarbonyl-Komplexe [^{99m}Tc(CO)₃(H₂O)₃]⁺, [Re(CO)₃(H₂O)₃] oder [NEt₄]₂[ReBr₃(CO)₃] in einer Mischung aus Wasser und Methanol erfolgen (vgl. Reaktionsschemata 3 und 4), wobei die Reaktionslösung für einen geeigneten Zeitraum, z.B. im Minuten- bis Stundenbereich, auf eine geeignete Temperatur von z.B. 50°C bis 60°C erhitzt wird. Gegebenenfalls kann zur Komplexierung noch eine Base wie Triethylamin (NEt₃) hinzugesetzt werden. Die Umsetzung in Gegenwart einer Hilfsbase wie NEt₃ erfolgt üblicherweise bei 50 °C (vgl. Beispiel [NEt₄]₂[ReBr₃(CO)₃]). Die Umsetzung mit den anderen oben genannten Metallionen kann z.B. in wässriger Kochsalzlösung oder einem Gemisch von Wasser mit einem organischen Lösungsmittel wie Ethanol, Ethylenglycol, Propylenglycol, DMSO oder DMF bei Raumtemperatur oder erhöhter Temperatur erfolgen. Der pH-Wert wird dabei üblicherweise mit einem geeigneten Puffersystem auf einem Wert zwischen 7 und 9 eingestellt. Typischerweise erfolgt die Komplexierung des Metallions bei einer Temperatur von 50°C.

Eine rein illustrative Verbindung, in der ein solcher Komplex vorkommt, ist eine Verbindung der Formel (III-a), die hier in der vom Träger T abgespaltenen Form dargestellt wird, und in der M Re oder ^{99m}Tc ist.

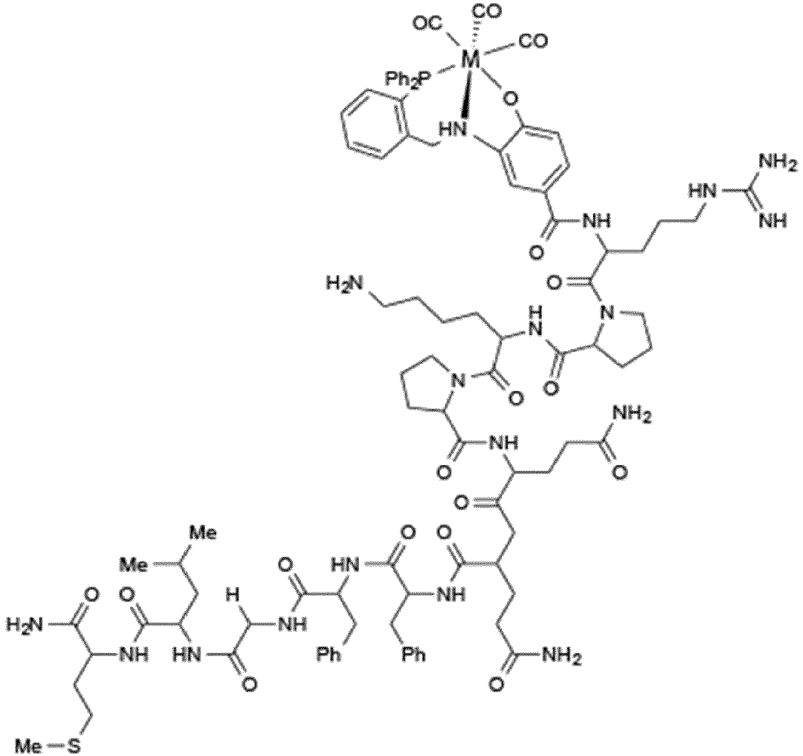

In dem Verfahren der Erfindung kann, wie oben bereits erwähnt, das Peptid Pep mittels eines Festphasensyntheseverfahrens, vorzugsweise eines automatisierten Verfahrens zur Festphasenpeptidsynthese hergestellt werden. Die Reaktionsbedingungen für Peptidfestphasensynthese (nach Merrifield) sind etabliert und dem Fachmann auf dem Gebiet der Peptidchemie bekannt. Die Verbindungen der Formeln (I) und (II) können daher ebenfalls vorzugsweise mit Hilfe eines automatisierten Verfahren zur Festphasenpeptidsynthese, bei dem das mit dem Metall M über die Bindungsgruppe BG komplexierte Peptid an den festen Träger kovalent gebunden wird, hergestellt werden. Die metallkomplexierte Verbindung, die durch Spaltung der kovalenten Bindung zwischen dem Polypeptid Pep und dem Träger T der Verbindung der Formel (I) hervorgeht, kann allgemein mit der Formel

H₂N-Pep-BG-M(L)ₙ

beschrieben werden, wobei L einen geeigneten Liganden L dargestellt und n eine Zahl zwischen 2 bis 4, in Abhängigkeit vom Metall M und der Zähnigkeit des Bindungsgruppe BG darstellt.

Bei der Spaltung der Verbindung der Formeln T-Pep-BG-M(Y1)(Y2)(Y3) (IIa), T-Pep-BG-M(Y1)(Y2) (IIb) oder T-Pep-BG-M(Y1) (IIc), resultieren die freien metallkomplexierten Verbindungen der Formeln H₂N-Pep-BG-M(Y1)(Y2)(Y3) (IIIa), H₂N-Pep-BG-M(Y1)(Y2) (IIIb) oder H₂N-Pep-BG-M(Y1) (IIIc).

Im Einklang mit dieser Offenbarung ist die Erfindung ebenfalls gerichtet auf die Verwendung einer Verbindung der Formel (I) oder der Formeln (IIa), (IIb) oder (IIc) zur Herstellung eines bildgebenden Reagenzes, wobei das Reagenz ein aus ^{99m}Tc, ¹⁸⁶Re, ¹⁸⁸Re, ⁹⁰Y, ⁸⁶Y, ¹⁷⁷Lu, ⁶⁸Ga, ⁶⁷Cu, ⁶⁴Cu, ⁶⁷Ga, ⁸⁹Zr, ¹⁵³Sm oder ¹¹¹In ausgewähltes Metall umfasst. Die Herstellung des bildgebenden Reagenzes umfasst dabei die Abspaltung der metall-komplexierten Verbindung vom festen Träger. Das so erhaltene bildgebende Reagenz wird üblicherweise in einem bildgebenden Verfahren wie Einzelphotonen-Emissionscomputertomographie (SPECT, Single Photon Emission Computed Tomography) oder Szintigraphie eingesetzt.

Die Erfindung ist ferner gerichtet auf die Verwendung einer Verbindung der Formel (I) zur Herstellung eines Strahlungstherapiemittels, wobei das Strahlungstherapiemittel ein aus ¹⁸⁶Re, ¹⁸⁸Re, ⁹⁰Y, ⁸⁶Y,¹⁷⁷Lu, ⁶⁸Ga, ⁶⁷Cu, ⁶⁴Cu, ⁶⁷Ga, ⁸⁹Zr, ¹⁵³Sm oder ¹¹¹In ausgewähltes Metall umfasst. Die Herstellung des Strahlungstherapiemittels umfasst dabei die Abspaltung der metall-komplexierten Verbindung vom festen Träger.

Die Erfindung ist auch gerichtet auf die Verwendung einer Verbindung der Formel (I) zum Einsatz eines damit hergestellten bildgebenden Reagenzes in einem bildgebenden Verfahren. Dabei dient das bildgebenden Reagenz beispielsweise zum Einsatz in Einzelphotonen-Emissionscomputertomographie (SPECT, Single Photon Emission Computed Tomography).

Die Erfindung ist ferner auch noch auf einen Kit zur Herstellung eines Radiopharmazeutikums oder einer radiodiagnostischen Zusammensetzung gerichtet, wobei das Radiopharmazeutikum oder die radiodiagnostische Zusammensetzung eine Verbindung der Formeln (IIIa), (IIIb) or (IIIc) umfasst, wobei der Kit eine Verbindung der Formel (I) umfasst und ein Reagenz, das ein Metall M umfasst, das aus ^{99m}Tc, ¹⁸⁶ Re, ¹⁸⁸ Re, ⁹⁰Y, ⁸⁶Y, ¹⁷⁷Lu, ⁶⁸Ga, ⁶⁷Cu, ⁶⁴Cu, ⁶⁷Ga, ⁸⁹Zr, ¹⁵³Sm und ¹¹¹In ausgewählt wird, um eine Verbindung der Formeln (III) zu bilden. Dabei kann in dem Kit das das Metall M umfassende Reagenz ein Vorläufer der Gruppe M(Y1)(Y2)(Y3) sein, wobei in der Gruppe M(Y1)(Y2)(Y3) Y1, Y2 und Y3 jeweils unabhängig voneinander CO oder Cl sind, oder eines von Y1, Y2 oder Y3 O ist und die beiden anderen unabhängig voneinander CO oder Cl sind. Alternativ dazu kann das Metallion auch durch Wassermoleküle komplexiert sein.

Die Erfindung wird weiterhin durch die nachfolgenden Beispiele und die beigefügten Figuren veranschaulicht. Dabei zeigen
**Fig. 1** die Festphasenmolekülstruktur von [Re(CO)₃(PHOSA)](Re-1),
**Fig.2** Chromatogramme für die HPLC-Reinigung der Komplexe Re-1 (UV) und Tc-1 (γ),
**Fig. 3** ein Chromatogramm der HPLC-Reinigung des Rohprodukts des Festphasensynthese von Substanz P (SP),
**Fig. 4** ein ³¹P-NMR-Spektrum des Rohprodukts des Festphasensynthese von Substanz P (SP),
**Fig. 5** ein Chromatogramm der HPLC-Reinigung des Rohprodukts der Festphasen-Markierung des Substanz P Analogons SP-PHOSA mit Re [Re(CO)₃(SP-PHOSA)] bzw. ^{99m}Tc [^{99m}Tc(CO)₃(SP-PHOSA)], und
**Fig. 6** ein ³¹P-NMR-Spektrum des Rohprodukts des markierten [Re(CO)₃(SP-PHOSA)].
**Fig. 7** den zeitlicher Verlauf der Bioverteilung des ^{99m}Tc-markierten Substanz P-Analogon in einer gesunden Maus, die durch *in-vivo*-Bildgebung mit einem Kleintier-SPECT/CT-System erhalten wurde,
**Fig. 8** ein Ganzkörper-Bild der Verteilung des ^{99m}Tc-markierten Substanz P-Analogon in der Maus 1 Stunde nach Injektion, wobei eine starke Ansammlung der Verbindung in der Lunge und in geringerem Maße im Darm deutlich nachweisbar ist.

### BEISPIELE

### Ligandensynthese:

Die Umsetzung von (2-Formylphenyl)diphenylphosphin bei 70 °C mit 3-Amino-4-hydroxybenzoesäure in Ethanol ergibt PHOSI als gelben Farbstoff. Die Iminfunktion von PHOSI kann unterschiedlichen Reaktionen wie Solvolyse oder Metall-induzierte Dissoziation unterworfen sein. Zur Verbesserung der chemischen Stabilität und physikalische Flexibilität des Liganden-Systems wurde PHOSI mit NaBH4 in siedendem Ethanol umgesetzt, um den Iminrest zum entsprechenden sekundären Amin zu reduzieren. Das resultierende bifunktionelle Phosphin PHOSA wurde in hoher Ausbeute isoliert und an der Luft bei 4°C für mehrere Wochen gelagert (Schema 1).

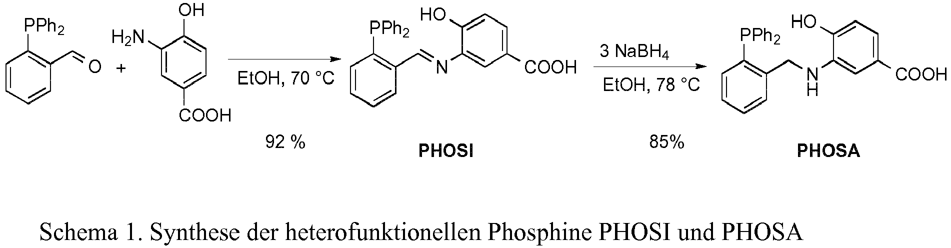

PHOSI und PHOSA wurden beide vollständig mittels NMR- und IR-Spektroskopie sowie Massenspektrometrie charakterisiert. ¹H-NMR-Spektren von PHOSI und PHOSA weisen breite Peaks bei 12,57 ppm und 12,18 ppm, die den Carbonsäure-Resten-COOH zugeordnet wurden, auf. Der Iminrest zeigt ein Dublett bei 9,23 ppm im ¹H-NMR-Spektrum von PHOSI, das erwartungsgemäß im Spektrum von PHOSA fehlt. Beide Verbindungen zeigen Resonanzen bei negativen chemischen Verschiebungen (δ_{PHOSI} = -13,8 ppm und δ_{PHOSA} = - 17,1 ppm) in ihren ³¹P NMR-Spektren, die im für Arylphosphine erwarteten Bereich liegen.

### Synthese der Komplexe [M(CO)₃(PHOSA)](M = Re, ^{99m}Tc)

Der Rhenium (I)-Komplex [NEt₄]₂[ReBr₃(CO)₃] (R. Alberto, R. Schibli, A. Egli, P. A. Schubiger, W. A. Hermann, G. Artus, U. Abram, T. A. Kaden, J, Organomet. Chem. 1995, 493, 119) reagiert mit PHOSA in Methanol bei moderaten Temperaturen. Die Hilfsbase NEt₃ wurde hinzugegeben, um die vollständige Koordination des Liganden durch gleichzeitige Entfernung der Brom-Liganden und Deprotonierung des Hydroxylrests zu erleichtern. Die Entfernung der Brom-Liganden kann durch Zugabe von AgNO₃ (3 Äq) erreicht werden, wobei die Präzipitation von AgBr die treibende Kraft für die Bildung von **Re-1** ist (Schema 2). Die Kristallisation von **Re-1** aus der gesättigten Lösung in Methanol bei -10 °C liefert farblose Plättchen von **Re-1,** die mit Einkristall-Röntgen-Analyse charakterisiert wurden.

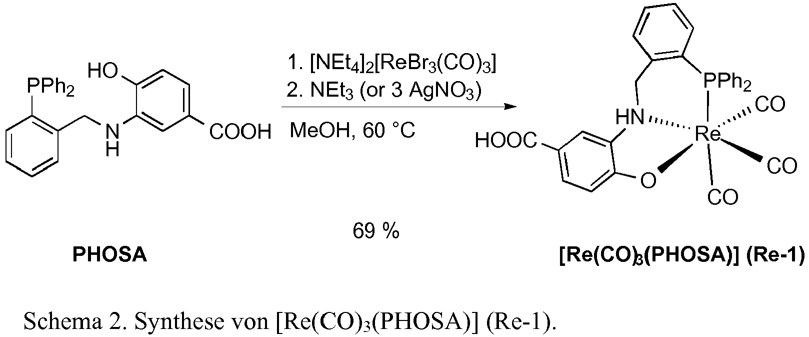

Das ³¹P NMR-Spektrum von Re-1 bestätigt die Koordination des Phosphoratoms mit Rhenium durch ein Singulett bei 5,2 ppm, das eine erhebliche Tieffeldverschiebung (22,9 ppm) im Vergleich zum unkoordinierten PHOSA aufweist. Im IR-Spektrum von **Re-1** erscheinen starke υ(CO) Streckschwingungen bei 2033, 1944 und 1894 cm⁻¹, was im Einklang mit anderen Komplexen steht, die die *fac*-[Re(CO)₃]⁺-Einheit enthalten (R. Schibli, K. V. Katti, W. A. Volkert, C. L. Barnes, Inorg. Chem. 2001, 40, 2358, bzw. S. Alvez, A. Paulo, J. D. G., Correia, A. Domingos, I. Santos, J. Chem. Soc., Dalton Trans. 2002, 4714).

Die Molekülstruktur von **Re-1** wurde durch Einkristall-Röntgenbeugung **(****Fig.1****)** ermittelt. Das Re-Atom ist sechsfach koordiniert und zeigt eine verzerrte oktaedrische Koordinationsgeometrie. Der einfach negativ geladene Ligand PHOSA besetzt eine Dreiecksfläche des Polyeders und die drei verbleibenden Koordinationsstellen werden durch endständige Carbonyl-Liganden besetzt. Der Carboxylrest ist außerhalb der Koordinationssphäre angeordnet, was eine ideale Konfiguration für Biokonjugations-Zwecke darstellt.

Um die Verwendung eines Liganden wie PHOSA in Radiomarkierungsverfahren zu zeigen, wurde PHOSA mit [^{99m}Tc(CO)₃(H₂O)₃]⁺ (401,7 MBq) in einer Mischung aus H₂O/MeOH (1:1) umgesetzt (Schema 3). Die Reaktionslösung wurde bei 60°C für 20 min erhitzt und die radiochemische Ausbeute und Reinheit wurde mittels HPLC untersucht. Der nicht radioaktive Komplex Re-1 wurde als Referenz verwendet.

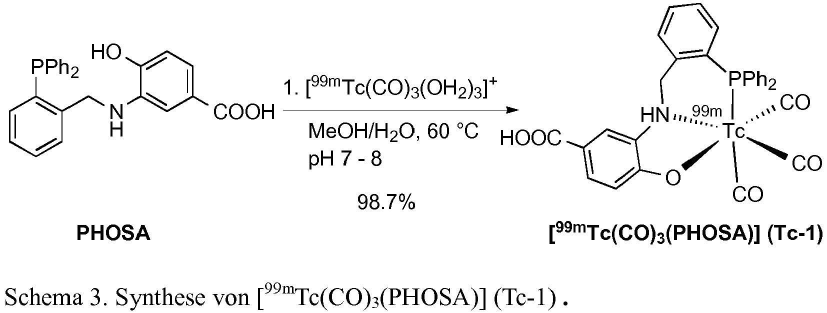

**Fig.2** zeigt die Chromatogramme der Komplexe Re-1 (UV) und Tc-1 (γ). Die Retentionszeiten der beiden Verbindungen sind erwartungsgemäß sehr ähnlich (t_{R} = 25,7 (Re-1) und 26,6 (Tc-1)). Wie in Fig. 2 gezeigt, beträgt die radiochemische Ausbeute der Reaktion nach 20 min Inkubation [^{99m}Tc(CO)₃(H₂O)₃]⁺ (pH 7-8) > 98%. Die relativ kurze Reaktionszeit (20 min), die eine hohe spezifische Aktivität ergibt, die nahezu quantitative Reaktionsausbeute sowie der bifunktionelle Koordinationsmodus von PHOSA bieten eine ideale Möglichkeit zur Biokonjugation sowie für ^{99m}Tc-Markierungsexperimente.

### Festphasensynthese des Konjugats von SP mit PHOSA

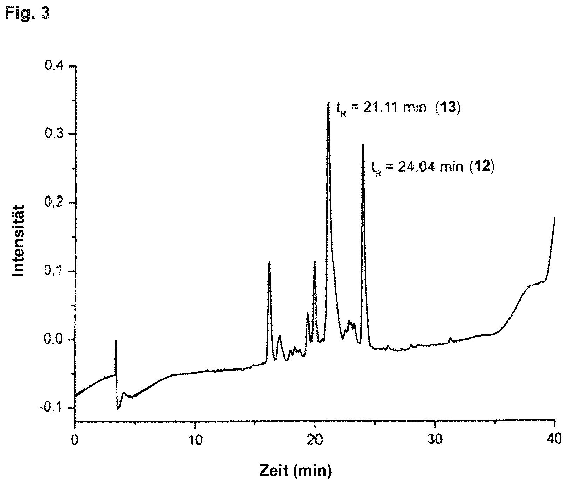

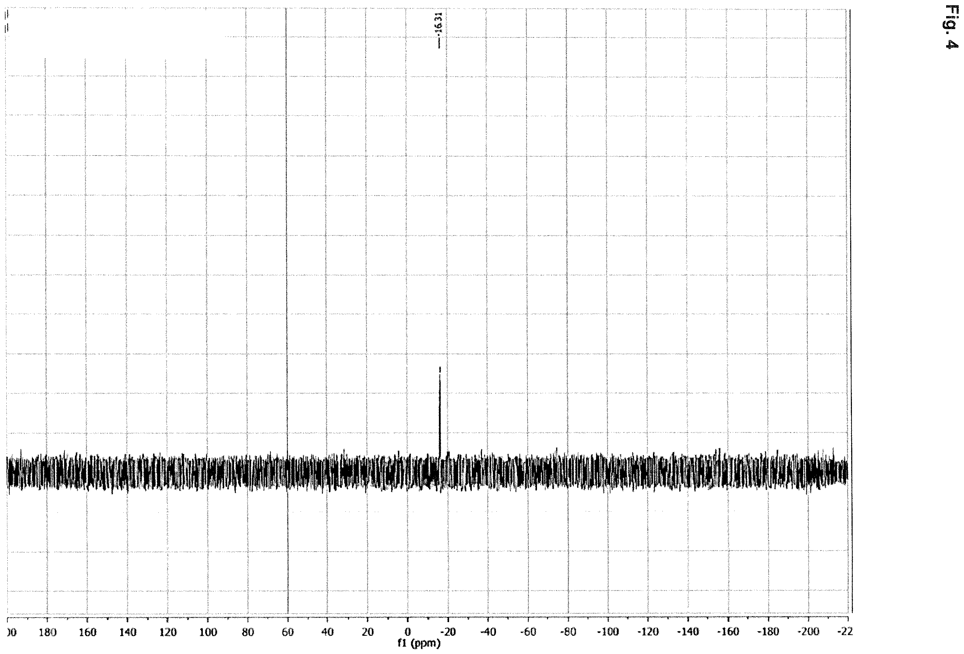

Das Neurotransmitter-Undecapeptid Pro Lys Pro Gln Gln Gly Phe Phe Leu Met (SEQ ID NO: 1), Substanz P (SP), wurde durch herkömmliche automatisierte Peptidsynthese synthetisiert, wobei NovaSyn®TGR-Harz als fester Träger verwendet wurde. Die an Harz gebundene SP wurde mit PHOSA (3 Äquivalente), das durch die Verwendung einer Mischung von DIC/HOAt (1:1) (DIC = N,N'-Diisopropylcarbodiimid; HOat = 1-Hydroxy-7-azabenzotriazol) präaktiviert worden war, in wasserfreiem DMF für 20 h bei Raumtemperatur umgesetzt. Um die Bildung von SP-PHOSA zu zeigen, wurden 1 mg der Harzkügelchen mit dem Spaltungscocktail TFA/TIS/H₂O/DTT (90:2.5:2.5:5) (TFA = Triflouressigsäure, TIS = Triisopropylsilan, DTT= Dithiothreitol) der mit Methionin enthaltenden Peptiden kompatibel ist, für 30 min (H. Huang, D. L. Rabenstein, J. Peptide Res. 1999, 53, 548) behandelt. Das Rohprodukt wurde durch HPLC und Massenspektrometrie analysiert. Die intensivsten Peaks im Chromatogramm (t_{R} = 21,11 min, **Fig. 3** sind der Masse des oxidierten SP-PHOSA [M+H +O]⁺, m/z = 1773,5 zugeordnet, das im MS(+ESI) Spektrum der gesammelten Fraktion nachgewiesen wurde. Dies Produkt kann von der Oxidation des Phosphoratoms von PHOSA unter den Spaltungsbedingungen stammen. Jedoch zeigt das ³¹P-NMR-Spektrum des Analyten anhand eines einzigen Peaks bei 35,07 ppm die Oxidation des Phosphinrestes an, während SP-PHOSA als Singulett bei -16,3 ppm erscheint (**Fig. 4**). Alle Versuche, die Oxidation von SP-PHOSA ohne den Schutz der Phosphinreste zu verhindern, blieben erfolglos.

### Festphasen-^{99m}Tc-Markierung des Substanz P Analogons SP-PHOSA und des entsprechenden Rheniumkomplex

Die für eine effiziente und quantitative Spaltung eines Peptids vom festen Träger übliche Zeit wird auf etwa 2 bis 3 h geschätzt (R. Schibli, et al, *supra,* S. Alvez, et al, *supra*). Dahingegen wurde, basierend auf den vorliegenden Untersuchungen der Erfinder, nach 1,5 h Aufbewahren der Harzperlen im Spaltungscocktail, die quantitative Bildung von Phosphinoxid beobachtet. Die Durchführung der Reaktion unter einer Inertgasatmosphäre konnte nicht zu einer deutlichen Verbesserung führen. Um dies zu verhindern, muss der Phosphinrest geschützt werden. Allerdings würden herkömmlichen Schutzgruppen von Phosphinen wie Borane (P. Pelion, Tetrahedron Lett. 1992, 33, 4451) die Synthese- und Isolierungsverfahren-Verfahren erschweren. Im Gegensatz dazu wird nach der Koordination des Phosphinrestes an ein Metallatom das Elektronenpaar des Phosphoratoms an Metall-Phosphor-Bindung beteiligt, womit der Phosphinrest nicht mehr einfach zu oxidieren ist. Daher wurde die Komplexbildungsreaktion auf dem festen Träger versucht und die Stabilität der SP-PHOSA-M (M = Re oder Tc)-Komplexe unter Spaltungsbedingungen untersucht (Schema 4).

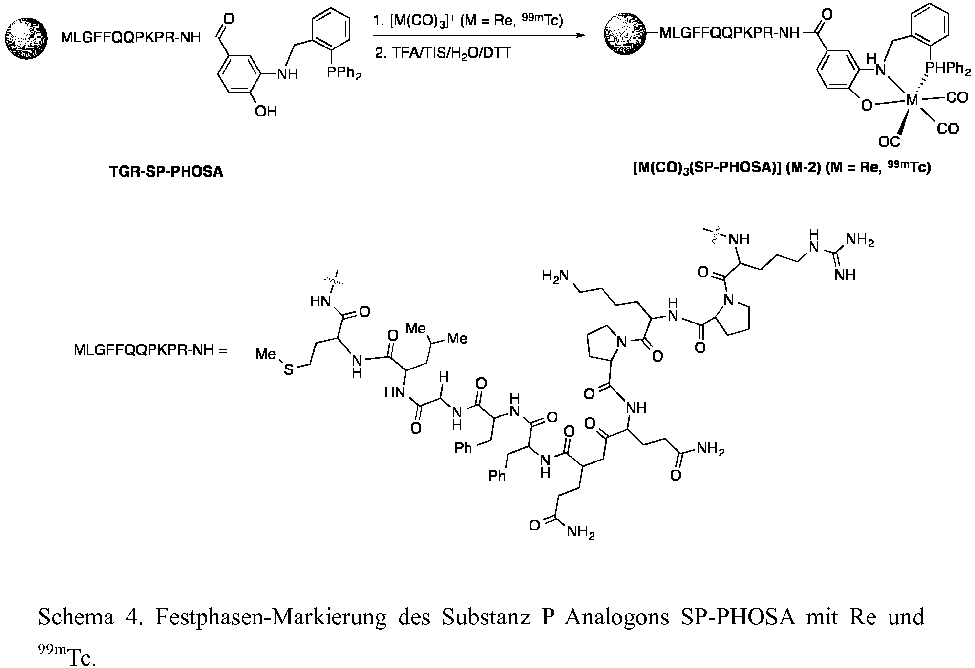

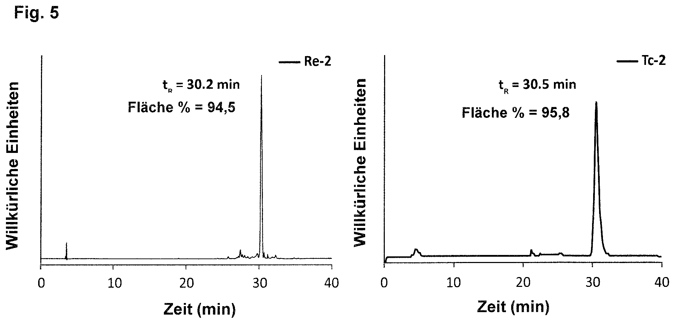

Festphasen-gebundenes SP Analog (TGR-SP-PHOSA) wurde mit [NEt₄]₂[ReBr₃(CO)₃] in Gegenwart der Hilfsbase NEt₃ in DMF bei 50 °C umgesetzt. Danach wurden die Harzkügelchen für 3 h mit dem Spaltungscocktail TFA/TIS/H₂O/DTT (90:2.5:2.5:5) behandelt. Die analytische HPLC-Spur des Rohprodukts zeigte einen einzigen Peak bei t_{R} = 30,2 min **(****Fig. 5****).** Das hochaufgelöste MS (+ESI)-Spektrum der gesammelten Fraktion zeigte die [M +2H]²⁺ und [M+3H]³⁺ Spezies bei m/z = 1014,395 bzw. 676,264. Beide Signale sind in vollem Einklang mit dem theoretischen für den Re-2-Komplex simulierten Isotopenverteilungsmuster.

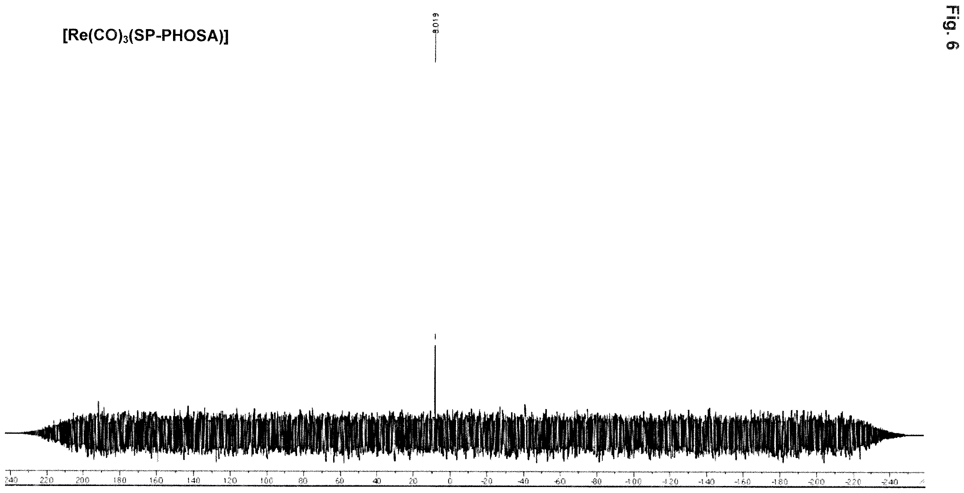

[Re(CO)₃(SP-PHOSA)] **(Re-2)** wurde mittels präparativer Umkehrphasen-HPLC isoliert und für 2 Tage gefriergetrocknet und dann mittels NMR und Massenspektrometrie charakterisiert. Das ³¹P-NMR-Spektrum von **Re-2 (****Fig. 6****)** zeigte eine einzelne Resonanz bei 8,02 ppm, die eine erwartete Tieffeldverschiebung im Vergleich zu SP-PHOSA (-16,3 ppm) darstellt. Die HPLC-Spur sowie die Massen- und ³¹P-NMR-Spektren zeigen klar, dass die Komplexbildungsreaktion auf dem festen Träger erfolgreich durchgeführt werden kann. Darüber hinaus ist die Reaktion sehr selektiv und vor allem bleibt der Rhenium-Komplex **Re-2** bleibt unter den Spaltungsbedingungen stabil.

Die Festphasenmarkierung des Analogons der Substanz P (SP-PHOSA) mit ^{99m}Tc-Markierung wurde durchgeführt, indem die Bedingungen für die Synthese von Re-2 wie folgt modifiziert wurden. TGR-SP-PHOSA wurde mit [^{99m}Tc(CO)₃(OH₂)₃]⁺ (0,5 ml, 608 MBq) in einer Mischung aus H₂O/DMF (1:3) bei 50 °C umgesetzt. Danach wurden die Harzperlen mehrfach mit DMF gewaschen. Die Aktivität aller gesammelten Lösungsmittel-Fraktionen von 40 MBq bestätigte, dass mehr als 91% der Radioaktivität von der Festphase aufgenommen wurden. Nach der Behandlung der Harzperlen mit dem Spaltungscocktail für annähernd 2 Stunden, wurde die Aktivität auf der Festphase sowie des Überstands separat gemessen. Die Aktivität betrug 311 MBq im Überstand und 113 MBq auf dem festen Träger. Die radiochemische Ausbeute der Markierungsreaktion bezogen auf die Aktivität des festen Trägers wurde nach mehreren Waschzyklen berechnet. Dementsprechend definiert die Aktivität der Harzkügelchen eine radiochemische Ausbeute von 76%, während die radiochemische Reinheit des Rohproduktes mit 95%, bezogen auf das radioaktive Spur der HPLC-Analyse, berechnet wurde. Die beiden Komplexe **Re-2** und **Tc-2** zeigten sehr ähnliche Retentionszeiten **(****Fig.5****),** wie es auch für die analogen Komplexe **Re-1** und **Tc-1** beobachtet wurde. Folglich bestätigten beide HPLC-Spuren, dass der heterofunktionelle Ligand PHOSA bemerkenswert stabile Komplexe sowohl mit Rhenium- und Technetium(I)-Tricarbonyl-Metallkernen bildet und die Komplexe (**Re-2** und **Tc-2**) selbst nicht unter den extremen Bedingungen bei der Spaltung vom Harz (reine Trifluoressigsäure) dissoziieren.

### Biodistribution: In vivo Einzelphotonen-Emissionscomputertomographie (SPECT imaging) und ex vivo Untersuchung

Das mittels des Verfahrens der vorliegenden Erfindung hergestellte ^{99m}Tc markierte Analogon von Substanz P (SP-PHOSA)^{99m}Tc wurde auf seine Eignung als Radiodiagnostikum (bildgebendes Reagenz) bzw. als Radiotherapeutikum untersucht. Dazu wurden gesunden Mäusen die Substanz P (SP-PHOSA)^{99m}Tc mittels Injektion verabreicht und die Verteilung der Verbindung nachfolgend untersucht. Für eine solche Untersuchung wurden typischerweise Markierungsansätze mit 60 MBq ^{99m}Tc hergestellt, in Phosphatpuffer gelöst und in die Schwanzvene injiziert.

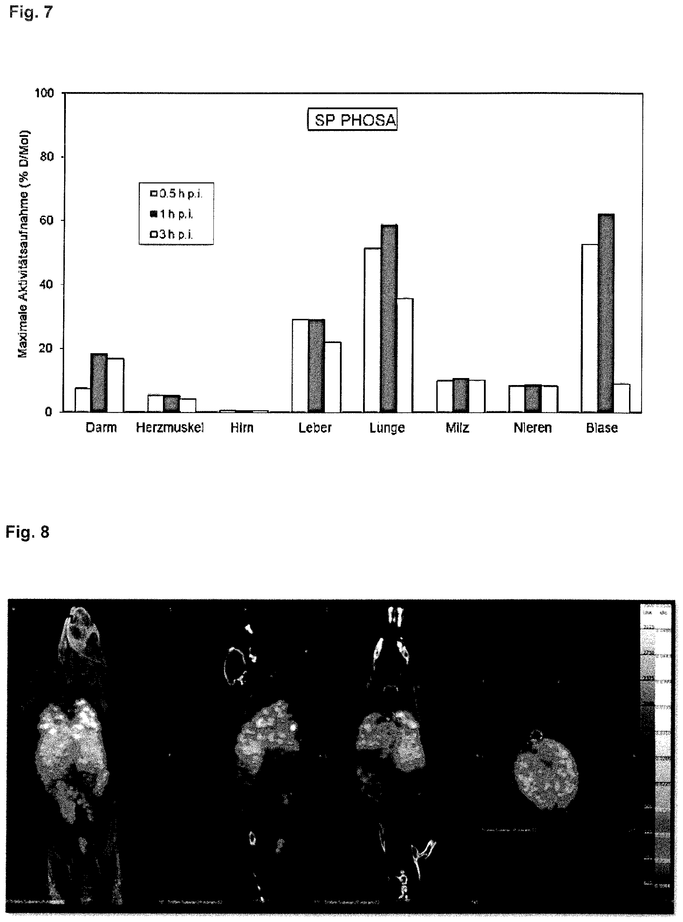

Die dabei festgestellte bemerkenswert hohe Aufnahme und Retention des SP-Agonisten SP-PHOSA in der Lunge **(****Fig. 7****)** steht in Übereinstimmung mit früheren Befunden beim Menschen mit Hilfe der Positronen-Emissions-Tomographie mit dem niedermolekularen Substanz P-Antagonist ¹⁸F-SPA-RQ [Sprague DR, Chin FT, Liow JS, Fujita M, Burns HD, Hargreaves R, et al. Human biodistribution and radiation dosimetry of the tachykinin NK1 antagonist radioligand [18F]SPA-RQ: comparison of thin-slice, bisected, and 2-dimensional planar image analysis. J Nucl Med. 2007;48:100-7.5]. In Anbetracht der geringen Gewebedichte der Lunge im Vergleich zu anderen Organen deutet dies auf eine extrem hohe Expression von spezifischen Bindungsstellen, vermutlich NK 1-Rezeptoren, in der Lunge hin. SP-PHOSA sammelte sich nicht im Gehirn an. Die sehr geringe im interessierenden Bereich des Gehirns gemessene Tracer-Konzentration stellt am ehesten Aktivität im Blutvolumen des Gehirns als im Hirngewebe dar. Dies deutet auf eine Undurchlässigkeit der Blut-Hirn-Schranke für den getesteten Tracer hin, in klarem Gegensatz zur Verbindung 18F-SPA-RQ, die eine hohe Aufnahme im Gehirn von Menschen, Primaten und Nagetieren [Sprague DR et al, supra; Haneda E, Higuchi M, Maeda J, Inaji M, Okauchi T, Ando K, et al. In vivo mapping of substance P receptors in brains of laboratory animals by high-resolution imaging systems. Synapse. 2007; 61:205-15.6] zeigte. Andere Studien fanden die Blut-Hirn-Schranke ebenfalls undurchlässig für einen In¹¹¹ markierten Substanz P-Agonisten [Breeman WA, VanHagen MP, Visser-Wisselaar HA, van der Pluijm ME, Koper JW, Setyono-Han B, et al. In vitro and in vivo studies of substance P receptor expression in rats with the new analog [indium111-DTPA-Argl]substance P. J Nucl Med. 1996;37:108-17]. Die durch SPECT-Untersuchungen bestimmte Organ-Bioverteilung **(****Fig. 8****)** war in guter Übereinstimmung mit den Neurokinin-Rezeptor-1-Boten-RNA-Spiegeln, die in verschiedenen Geweben der Ratte nachgewiesen worden waren [McCarson KE. Central and peripheral expression of neurokinin-1 and neurokinin-3 receptor and substance P-encoding messenger RNAs: peripheral regulation during formalin-induced inflammation and lack of neurokinin receptor expression in primary afferent sensory neurons. Neuroscience. 1999;93:361-70]. Diese Studien zeigten jedoch auch eine starke Expression des Rezeptors im Gehirn. Dies konnte jedoch aufgrund der Undurchlässigkeit der Blut-Hirn-Schranke für den verabreichten Tracer nicht durch SPECT reproduziert werden.

Die Ergebnisse der *in vivo* SPECT-Aufnahmen wurden von den *Ex-vivo-*Messungen bestätigt, d.h. es existierte eine gute Korrelation zwischen der Aufnahme bei der 3 h SPECT Messung und den Ex-vivo-Ergebnissen. Das beweist die Wirksamkeit der Kleintier-SPECT-Technologie für eine präzise *in-vivo* Quantifizierung der Traceraufhahme in verschiedenen Organen der Maus (Finucane CM, Murray I, Sosabowski JK, Foster JM, Mather SJ. Quantitative Accuracy of Low-Count SPECT Imaging in Phantom and In Vivo Mouse Studies. Int J Mol Imaging. 2011). Auch diese Daten zeigen die Eignung des Verfahrens und der Verbindungen der vorliegenden Erfindung zur Herstellung von bildgebenden Reagenzien und Radiopharmaka.

## Patentansprüche

1. Eine Verbindung der Formel (I)
T-Pep-BG (I)
wobei T ein fester Träger ist;
Pep ein kovalent an T gebundenes Polypeptid ist, wobei das Polypeptid eine Aminosäuresequenz von mindestens 2 Aminosäuren, vorzugweise eine Aminosäuresequenz mit 3 bis 200 Aminosäuren aufweist, und BG eine (nicht-komplexierte) Bindungsgruppe für ein Metall M ist, das aus der Gruppe ausgewählt wird, die aus ^{99m}Tc, ¹⁸⁶Re, ¹⁸⁸Re, ⁹⁰Y, ⁸⁶Y, ¹⁷⁷Lu, ⁶⁸Ga, ⁶⁷Cu, ⁶⁴Cu, ⁶⁷Ga, ⁸⁹Zr, ¹⁵³Sm und ¹¹¹In besteht,
wobei BG ausgewählt wird aus gegebenenfalls substituierten Aryl-Phosphanderivaten oder gegebenenfalls substituierten Heteroaryl-Phosphanderivaten der Formel (IIa)
P(R⁵)₃ (IIa),
wobei R⁵ gegebenenfalls substituiertes Aryl oder gegebenenfalls substituiertes Heteroaryl ist, wobei der Substituent von Aryl oder Heteroaryl ausgewählt wird aus R⁴-C₁-C₆ Alkyl, R⁴-NH-(C₁-C₆)Alkyl, R⁴-N=C-, R⁴-(C₁-C₆)Alkyl-NH-NH-(C₁-C₆)Alkyl, wobei R⁴ ausgewählt wird aus Aryl, Heteroaryl oder Heterocyclyl, die gegebenenfalls alle mit einem oder zwei Substituenten R⁶ substituiert sind, wobei R⁶ aus OH-(C₁-C₆) Alkyl, OH, NH₂, und (C₁-C₆)Alkoxy ausgewählt wird,
und wobei zumindest einer der Reste R⁵ mit einem Substituenten R¹ versehen ist, der aus -CO-, -S-, -NH- oder -O- ausgewählt wird,
wobei unter Alkyl auch Alkenyl, Alkinyl, und gesättigtes und teilweise ungesättigtes Cycloalkyl und Cycloalkylalkyl zu verstehen ist, und wobei die Gruppe BG mittels des Substituenten R¹ mit einer reaktiven Seitenkette einer Aminosäure von Pep oder der Alpha-Aminogruppe oder der Carboxylgruppe einer Aminosäure von Pep kovalent verbunden ist.

2. Die Verbindung der Formel (I) nach Anspruch 1, wobei BG eine Gruppe der Formel (A) oder (B) oder (C) oder (D) oder (E) oder (F) oder (G) ist 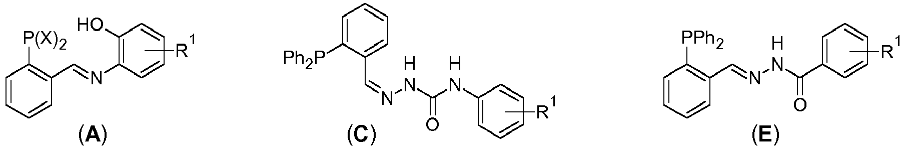 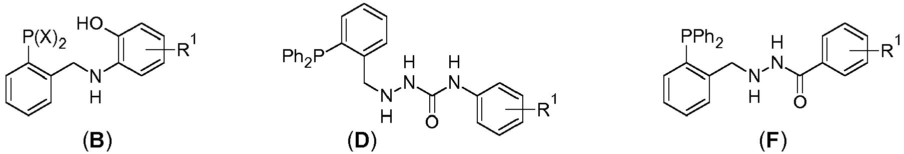 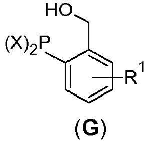 wobei in Formeln (A), (B), (C), (D), (E), (F) oder (G) die Gruppe BG mittels des Substituenten R¹ kovalent mit einer Seitenkette einer Aminosäure von Pep oder mit der Alpha-Aminogruppe oder der Carboxylgruppe einer Aminosäure von Pep verbunden ist,
wobei
R¹ aus -CO-, -S-, -NH-, oder -O- ausgewählt ist;
X, jeweils unabhängig voneinander ausgewählt wird aus einer gegebenenfalls substituierten Arylgruppe, wobei die Substituenten ausgewählt werden aus (C₁-C₆)Alkyl, (C₁-C₆) Alkoxy, Hydroxy- (C₁-C₆) Alkyl, oder X eine Gruppe der Formeln (a) oder (b) 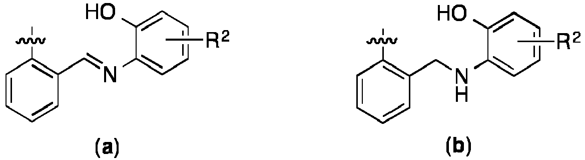
ist, wobei in den Formel (a) und (b)
R² H, (C₁-C₆)Alkyl, (C₁-C₆)Alkoxy, Halogen, OH, NH₂, -CN, Aryl, SH, oder
-S(C₁-C₆)-Alkyl ist,
wobei unter Alkyl auch Alkenyl, Alkinyl und gesättigtes und teilweise ungesättigtes Cycloalkyl und Cycloalkylalkyl zu verstehen ist.

3. Die Verbindung der Formel (I) gemäß Anspruch 2, wobei R¹ in meta-Position zu der NH-Gruppe desselben Arylrings angeordnet ist.

4. Die Verbindung der Formel (I) gemäß einem der Ansprüche 2 oder 3, wobei R¹ -COist.

5. Die Verbindung der Formel (I) gemäß einem der Ansprüche 2 bis 4, wobei X Phenyl oder gegebenenfalls substituiertes Phenyl ist.

6. Die Verbindung der Formel (I) gemäß einem der Ansprüche 1 bis 5, wobei Pep 3 bis 50, 3 bis 40, 3 bis 30, 3 bis 25, 3 bis 22, 3 bis 20, 3 bis 17, 3 bis 15, oder 3 bis 10 Aminosäuren aufweist.

7. Die Verbindung der Formel (I) gemäß einem der vorhergehenden Ansprüche, wobei das Peptid aus der Gruppe ausgewählt wird, die aus
- Arg Pro Lys Pro Gln Gln Phe Phe Gly Leu Met (SEQ ID NO: 1) (Neuropeptid Substanz P) und Fragmente davon
- GNGRG (SEQ ID NO: 2)
- (GNGRG)₂KNK (SEQ ID NO: 3)
- Formyl MLF (der Prototyp von Formylpeptiden, die Neutrophilrezeptoren binden)
- (VGVAPG)₃ (SEQ ID NO: 4)
- (VPGVG) (SEQ ID NO: 5)
- RALVDTLKFVTQAEGAK (SEQ ID NO:6)
- RALVDTEFKVKQEAGAK (SEQ ID NO: 7)
- PLARITLPDFRLPEIAIP (SEQ ID NO: 8)
- GQQHHLGGAKAGDV (SEQ ID NO: 9)
- PLYKKIIKKLLES (SEQ ID NO: 10)
- LRALVDTLK (SEQ ID NO: 11)
- GGGLRALVDTLK (SEQ ID NO: 12)
- GGGLRALVDTLKFVTQAEGAK (SEQ ID NO: 13)
- GGGRALVDTLKALVDTL (SEQ ID NO: 14)
- GHRPLDKKREEAPSLRPAPPPISGGGYR (SEQ ID NO: 15)
- PSPSPIHPAHHKRDRRQ (SEQ ID NO: 16)
- GGGFD (SEQ ID NO: 17)
- YWDKTFT (SEQ ID NO: 18)
- SYNRGDSTC (SEQ ID NO: 19)
- GGGLRALVDTLK (SEQ ID NO: 20)
- GCGGGLRALVDTLK (SEQ ID NO: 21)
- GCYRALVDTLKFVTQAEGA (SEQ ID NO: 22)
- GC(VGVAPG)₃ (SEQ ID NO: 23)
- Neuropeptid Y (36AS)
- Somastin (92 AS)
- Cholecystokinin (bis 95AS)
- Pankrezymin (bis 95AS)
- Bombesin (14 AS)
- Galanin (30AS)
- Bradykinin (9AS)
- Angiotensin II (8AS)
- Calcitonin Gen-Related Peptide (CGRP) (37AS) und besteht.

8. Die Verbindung der Formel (I) gemäß einem der vorhergehenden Ansprüche, wobei die Bindungsgruppe BG an die alpha-Aminogruppe des N-terminalen Aminosäurerests von Pep oder an eine Seitenkette eines innerhalb von Pep angeordneten Aminosäurerests gebunden ist.

9. Die Verbindung der Formel (I) gemäß einem der vorhergehenden Ansprüche, wobei der Träger T ein Harz ist, das als fester Träger in einem automatisierten Festphasenpeptidsynthese-Verfahren geeignet ist.

10. Verwendung einer wie in den Ansprüchen 1-9 definierten Verbindung der Formel (I) zur Herstellung eines bildgebenden Reagenzes, wobei das Reagenz ein aus ^{99m}Tc, ¹⁸⁶ Re, ¹⁸⁸Re, ⁹⁰Y, ⁸⁶Y, ¹⁷⁷Lu, ⁶⁸Ga, ⁶⁷Cu, ⁶⁴Cu, ⁶⁷Ga, ⁸⁹Zr, ¹⁵³Sm und ¹¹¹In ausgewähltes Metall umfasst.

11. Verwendung einer wie in den Ansprüchen 1-9 definierten Verbindung der Formel (I) zur Herstellung eines Strahlungstherapiemittels, wobei das Strahlungstherapiemittel ein Metall umfasst, das aus ⁹⁹Tc, ¹⁸⁶Re, ¹⁸⁸Re, ⁹⁰Y, ⁸⁶Y,¹⁷⁷Lu, ⁶⁸Ga, ⁶⁷Cu, ⁶⁴Cu, ⁶⁷Ga, ⁸⁹Zr, ¹⁵³Sm und ¹¹¹In ausgewählt wird.

12. Verwendung gemäß Anspruch 10, wobei das bildgebende Reagenz zur Verwendung in einem bildgebenden Verfahren eingesetzt wird, wobei das bildgebende Verfahren vorzugsweise Einzelphotonen-Emissionscomputertomographie (SPECT, Single Photon Emission Computed Tomography) oder Szintigraphie ist.

13. Verfahren zur Herstellung einer Verbindung der Formel (I)
T-Pep-BG (I)
wobei T, Pep und BG die in den Ansprüchen 1-9 angegebenen Bedeutungen haben, wobei das Verfahren umfasst:
a) Synthetisieren eines Polypeptids Pep, wobei Pep ein Polypeptid ist, das kovalent an den Träger T gebunden wird und eine Aminosäuresequenz mit 3 bis 200 Aminosäuren aufweist,
um eine Verbindung der Formel
T-Pep
zu erhalten;
b) Umsetzen von T-Pep mit einem Vorläufer BG' von BG, um eine Verbindung der Formel (I)
T-Pep-BG (I)
zu erhalten,
wobei BG eine (nicht komplexierte) Bindungsgruppe für ein Metall M ist, wobei das Metall M aus der Gruppe ausgewählt wird, die aus ^{99m}Tc, ¹⁸⁶Re, ¹⁸⁸Re, ⁹⁰Y, ⁸⁶Y, ¹⁷⁷Lu, ⁶⁸Ga, ⁶⁷Cu, ⁶⁴Cu, ⁶⁷Ga, ⁸⁹Zr, ¹⁵³Sm und ¹¹¹ besteht.

14. Verfahren nach Anspruch 13, das weiterhin umfasst:
c) Umsetzen der Verbindung der Formel (I) mit einem Reagenz, das ein Metall M umfasst, um eine Verbindung der Formel (II)
T-Pep-BG-M(Y1)(Y2)(Y3) (II)
zu bilden,
wobei Y1, Y2 und Y3 jeweils unabhängig voneinander CO oder Cl sind, oder eines von Y1, Y2 oder Y3 O ist und die beiden anderen unabhängig voneinander CO oder Cl sind, und
M aus ^{99m}Tc, ¹⁸⁶Re, ¹⁸⁸Re, ⁹⁰Y, ⁸⁶Y, ¹⁷⁷Lu, ⁶⁸Ga, ⁶⁷Cu, ⁶⁴Cu, ⁶⁷Ga, ⁸⁹Zr ¹⁵³Sm und ¹¹¹In ausgewählt wird; und
d) gegebenenfalls Spalten der Bindung zwischen T und Pep, um eine Verbindung der Formel (III)
H₂N-Pep-BG-M(Y1)(Y2)(Y3) (III)
zu erhalten.

15. Kit zur Herstellung eines Radiopharmazeutikums oder einer radiodiagnostischen Zusammensetzung, wobei das Radiopharmazeutikum oder die radiodiagnostische Zusammensetzung eine im Anspruch 14 definierte Verbindung der Formel (III) umfasst, wobei der Kit umfasst eine wie in den Ansprüchen 1-8 definierte Verbindung der Formel (I) und ein Reagenz, das ein Metall M umfasst, das aus ^{99m}Tc, ¹⁸⁶Re, ¹⁸⁸Re, ⁹⁰Y, ⁸⁶Y,¹⁷⁷Lu, ⁶⁸Ga, ⁶⁷Cu, ⁶⁴Cu, ⁶⁷Ga, ⁸⁹Zr ¹⁵³Sm und ¹¹¹In ausgewählt wird, um eine Verbindung der Formel (III) zu bilden.

## Claims

1. A compound of formula (I)
T-Pep-BG (I)
Wherein T is a solid support;
Pep is a polypeptide covalently bonded to T, wherein the polypeptide has an amino acid sequence of at least 2 amino acids, preferably an amino acid sequence of 3 to 200 amino acids, and BG is a (non-complexed) binding group for a metal M, said metal being selected from the group consisting of ^{99m}Tc, ¹⁸⁶Re, ¹⁸⁸Re, ⁹⁰Y, ⁸⁶Y, ¹⁷⁷Lu, ⁶⁸Ga, ⁶⁷Cu, ⁶⁴Cu, ⁶⁷Ga, ⁸⁹Zr, ¹⁵³Sm and ¹¹¹In,
wherein BG is selected from optionally substituted arylphosphane derivatives or optionally substituted heteroarylphosphane derivatives of formula (IIa)
P(R⁵)₃ (IIa),
wherein R⁵ is an optionally substituted aryl or an optionally substituted heteroaryl, wherein the substituent of the aryl or heteroaryl is selected from R⁴-(C₁-C₆-alkyl), R⁴-NH-(C₁-C₆-alkyl), R⁴-N=C-,R⁴-(C₁-C₆-alkyl)-NH-NH-(C₁-C₆-alkyl), wherein R⁴ is selected from aryl, heteroaryl, or heterocyclyl all of which are optionally substituted with one or two substituents R⁶, wherein R⁶ is selected from OH-(C₁-C₆-alkyl), OH, NH₂, and C₁-C₆-alkoxy,
and wherein at least one of the residues R⁵ is provided with substituents R¹ selected from -CO-, -S-, -NH-, or -O-, wherein alkyl is also understood to mean alkenyl, alkynyl, and saturated and partially unsaturated cycloalkyl and cycloalkylalkyl, and
wherein the group BG is covalently bonded by means of the substituent R¹ with a reactive side chain of an amino acid of Pep or the alpha-amino group or the carboxyl group of an amino acid of Pep.

2. The compound of formula (I) according to claim 1, wherein BG is a group of formula (A) or (B) or (C) or (D) or (E) or (F), or (G) 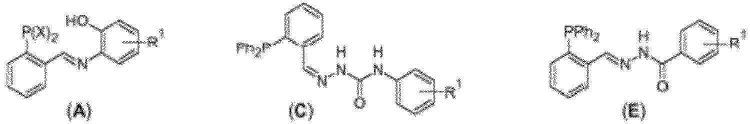 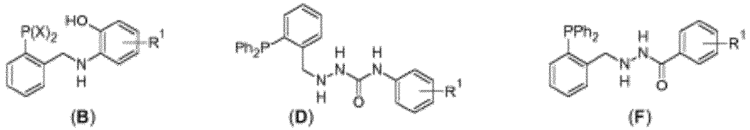 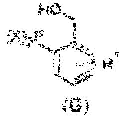
wherein in formulas (A), (B), (C), (D), (E), (F), or (G) the group BG is covalently bonded by means of the substituent R¹ with a side chain of an amino acid of Pep or with the alpha-amino group or the carboxyl group of an amino acid of Pep,
wherein
R¹ is selected from -CO- -S- -NH- or -O-;
X, is respectively independently from one another selected from an optionally substituted aryl group, wherein the substituents are selected from C₁-C₆-alkyl, C₁-C₆-alkoxy, hydroxy-(C₁-C₆-alkyl), or X is a group of formula (a) or (b), 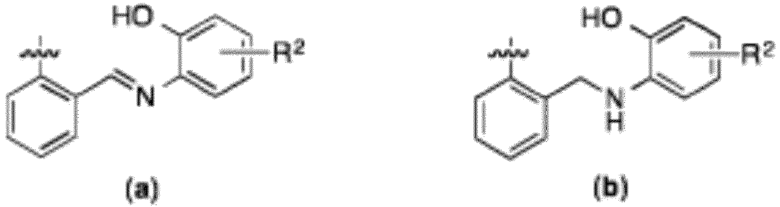
wherein in the formulas (a) and (b)
R² is H, C₁-C₆-alkyl, C₁-C₆-alkoxy, halogen, OH, NH₂,-CN, aryl, SH, or -S-(C₁-C₆-alkyl),
wherein alkyl is also understood to mean alkenyl, alkynyl, and saturated and partially unsaturated cycloalkyl and cycloalkylalkyl.

3. The compound of formula (I) according to claim 2, wherein R¹ is arranged in the meta-position to the NH group of the same aryl ring.

4. The compound of formula (I) according to one of the claims 2 or 3, wherein R¹ is -CO-.

5. The compound of formula (I) according to one of the claims 2 through 4, wherein X is phenyl or optionally substituted phenyl.

6. The compound of formula (I) according to one of the claims 2 through 5, wherein Pep has 3 to 50, 3 to 40, 3 to 30, 3 to 25, 3 to 22, 3 to 20, 3 to 17, 3 to 15, or 3 to 10 amino acids.

7. The compound of formula (I) according to one of the preceding claims, wherein the peptide is selected from the group consisting of
- Arg Pro Lys Pro Gln Gln Phe Phe Gly Leu Met (SEQ ID NO: 1) (neuropeptide substance P) and fragments thereof
- GNGRG (SEQ ID NO: 2)
- (GNGRG)₂KNK (SEQ ID NO: 3)
- Formyl MLF (the prototype of formyl peptides that bind neutrophil receptors)
- (VGVAPG)₃ (SEQ ID NO: 4)
- (VPGVG) (SEQ ID NO: 5)
- RALVDTLKFVTQAEGAK (SEQ ID NO:6)
- RALVDTEFKVKQEAGAK (SEQ ID NO: 7)
- PLARITLPDFRLPEIAIP (SEQ ID NO: 8)
- GQQHHLGGAKAGDV (SEQ ID NO: 9)
- PLYKKIIKKLLES (SEQ IDNO: 10)
- LRALVDTLK (SEQ IDNO: 11)
- GGGLRALVDTLK (SEQ ID NO: 12)
- GGGLRALVDTLKFVTQAEGAK (SEQ ID NO: 13)
- GGGRALVDTLKALVDTL (SEQ IDNO: 14)
- GHRPLDKKREEAPSLRPAPPPISGGGYR (SEQ ID NO: 15)
- PSPSPIHPAHHKRDRRQ (SEQ ID NO: 16)
- GGGFD (SEQ ID NO: 17)
- YWDKTFT (SEQ ID NO: 18)
- SYNRGDSTC (SEQ ID NO: 19)
- GGGLRALVDTLK (SEQ ID NO: 20)
- GCGGGLRALVDTLK (SEQ ID NO: 21)
- GCYRALVDTLKFVTQAEGA (SEQ ID NO: 22)
- GC(VGVAPG)₃ (SEQ ID NO: 23)
- Neuropeptide Y (36 AA)
- Somastin (92 AA)
- Cholecystokinin (up to 95 AA)
- Pancreozymin (up to 95 AA)
- Bombesin (14 AA)
- Galanin (30 AA)
- Bradykinin (9 AA)
- Angiotensin II (8 AA)
- Calcitonin gene-related peptide (CGRP) (37 AA).

8. The compound of formula (I) according to one of the preceding claims,
wherein the
binding group BG is linked to the alpha-amino group of the N-terminal amino acid residue of Pep or to a side chain of an amino acid residue arranged within Pep.

9. The compound of formula (I) according to one of the preceding claims, wherein the support T is a resin that is suitable as a solid support in an automated solid-phase peptide synthesis method.

10. A use of a compound of formula (I) as defined in claims 1-9 for the production of an imaging reagent, wherein the reagent comprises a metal selected from ^{99m}Tc, ¹⁸⁶Re, ¹⁸⁸Re, ⁹⁰Y, ⁸⁶Y, ¹⁷⁷Lu, ⁶⁸Ga, ⁶⁷Cu, ⁶⁴Cu, ⁶⁷Ga, ⁸⁹Zr, ¹⁵³Sm, and ¹¹¹In.

11. A use of a compound of formula (I) as defined in claims 1-9 for the production of a radiotherapy agent, wherein the radiotherapy agent comprises a metal selected from ^{99m}Tc, ¹⁸⁶Re, ¹⁸⁸Re, ⁹⁰Y, ⁸⁶Y, ¹⁷⁷Lu, ⁶⁸Ga, ⁶⁷Cu, ⁶⁴Cu, ⁶⁷Ga, ⁸⁹Zr, ¹⁵³Sm, and ¹¹¹In.

12. The use according to claim 10, wherein the imaging reagent is used for an application in an imaging method, wherein the imaging method is preferably single-photon emission computed tomography (SPECT) or scintigraphy.

13. A method for the production of a compound of formula (I)
T-Pep-BG (I)
wherein T, Pep, and BG have the same meaning as that given in claims 1-9, wherein the method comprises:
a) synthesis of a polypeptide Pep, wherein Pep is a polypeptide that is covalently linked to the support T and has an amino acid sequence with 3 to 200 amino acids,
to obtain a compound of the formula
T-Pep;
b) reaction of T-Pep with a BG precursor BG' to obtain a compound of formula (I)
T-Pep-BG (I),
wherein BG is a (non-complexed) binding group for a metal M, wherein the metal M is selected from a group that consists of ^{99m}Tc, ¹⁸⁶Re, ¹⁸⁸Re, ⁹⁰Y, ⁸⁶Y, ¹⁷⁷Lu, ⁶⁸Ga, ⁶⁷Cu, ⁶⁴Cu, ⁶⁷Ga, ⁸⁹Zr, ¹⁵³Sm, and ¹¹¹In.

14. The method according to claim 13, that further comprises:
c) reaction of the compound of formula (I) with a reagent that comprises a metal M to form a compound of formula (II)
T-Pep-BG-M(Y1)(Y2)(Y3) (II),
wherein Y1, Y2, and Y3 respectively independently from one another are CO or Cl, or one of Y1, Y2, or Y3 is O and the other two respectively independently from one another are CO or Cl, and
M is selected from ^{99m}Tc, ¹⁸⁶Re, ¹⁸⁸Re, ⁹⁰Y, ⁸⁶Y,¹⁷⁷Lu, ⁶⁸Ga, ⁶⁷Cu, ⁶⁴Cu, ⁶⁷Ga, ⁸⁹Zr, ¹⁵³Sm, and ¹¹¹In; and
d) optionally cleavage of the bond between T and Pep to obtain a compound of formula (III)
H₂N-Pep-BG-M(Y1) (Y2) (Y3) (III).

15. A kit for the production of a radiopharmaceutical or a radiodiagnostic composition, wherein the radiopharmaceutical or a radiodiagnostic composition comprises a compound of formula (III) as defined in claim 14, wherein the kit comprises a compound of formula (I) as defined in claims 1-8 and a reagent that comprises a metal selected from ^{99m}Tc, ¹⁸⁶Re, ¹⁸⁸Re, ⁹⁰Y, ⁸⁶Y, ¹⁷⁷Lu, ⁶⁸Ga, ⁶⁷Cu, ⁶⁴Cu, ⁶⁷Ga, ⁸⁹Zr, ¹⁵³Sm, and ¹¹¹In to form a compound of formula (III).

## Revendications

1. Composé de formule (I)
T-Pep-BG (I)
dans laquelle T est un support solide ;
Pep est un polypeptide lié par covalence à T, le polypeptide présentant une séquence d'acides aminés d'au moins 2 acides aminés, de préférence une séquence d'acides aminés comportant 3 à 200 acides aminés, et BG est un groupe de liaison (non complexé) pour un métal M qui est choisi dans le groupe qui est constitué par ^{99m}Tc, ¹⁸⁶Re, ¹⁸⁸Re, ⁹⁰Y, ⁸⁶Y, ¹⁷⁷Lu, ⁶⁸Ga, ⁶⁷Cu, ⁶⁴Cu, ⁶⁷Ga, ⁸⁹Zr, ¹⁵³Sm et ¹¹¹In,
BG étant choisi parmi des dérivés d'aryl-phosphane éventuellement substitués ou des dérivés d'hétéroaryl-phosphane éventuellement substitués de formule (IIa)
P(R⁵)₃ (IIa),
dans laquelle R⁵ est un aryle éventuellement substitué ou un hétéroaryle éventuellement substitué, le substituant de l'aryle ou de l'hétéroaryle étant choisi parmi le R⁴-alkyle(C₁-C₆), R⁴-NH-alkyle(C₁-C₆), R⁴-N=C-, R⁴-alkyl(C₁-C₆)-NH-NH-alkyle(C₁-C₆), où R⁴ est choisi parmi l'aryle, l'hétéroaryle et l'hétérocyclyle, qui sont tous éventuellement substitués par un ou deux substituants R⁶, R⁶ étant choisi parmi le OH-alkyle(C₁-C₆), le OH, le NH₂ et l'alcoxy en C₁-C₆,
et dans laquelle au moins l'un des radicaux R⁵ est porteur d'un substituant R¹ qui est choisi parmi -CO-, -S-, -NH- et -O-,
où par alkyle il faut entendre également alcényle, alcynyle, et cycloalkyle ou cycloalkylalkyle saturé ou partiellement insaturé, et
où le groupe BG est lié par covalence au moyen du substituant R¹ à une chaîne latérale réactive d'un acide aminé de Pep ou au groupe alpha-amino ou au groupe carboxy d'un acide aminé de Pep.

2. Composé de formule (I) selon la revendication 1, dans lequel BG est un groupe de formule (A) ou (B) ou (C) ou (D) ou (E) ou (F) ou (G) 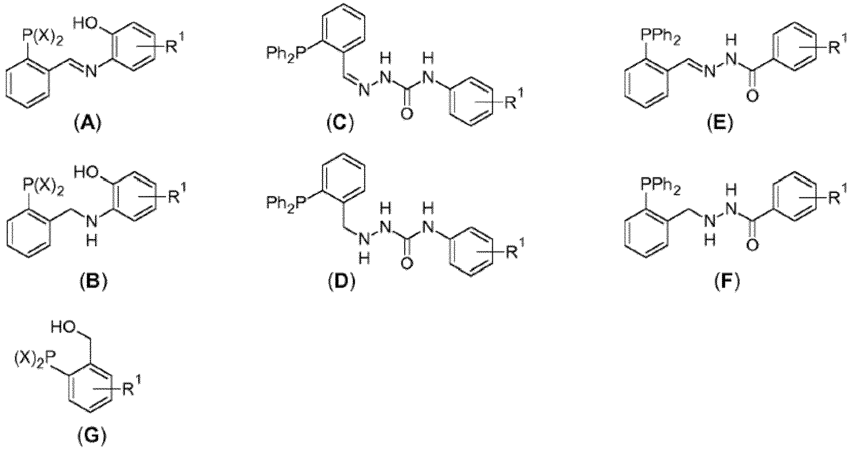
dans les formules (A), (B), (C), (D), (E), (F) et (G), le groupe BG étant lié par covalence au moyen du substituant R¹ à une chaîne latérale d'un acide aminé de Pep ou au groupe alpha-amino ou au groupe carboxy d'un acide aminé de Pep,
où
R¹ est choisi parmi -CO-, -S-, -NH-, et -O- ;
X est choisi, chaque fois indépendamment, parmi un groupe aryle éventuellement substitué, les substituants étant choisis parmi l'alkyle en C₁-C₆, l'alcoxy en C₁-C₆, l'hydroxy-alkyle(C₁-C₆), ou X est un groupe de formule (a) ou (b) 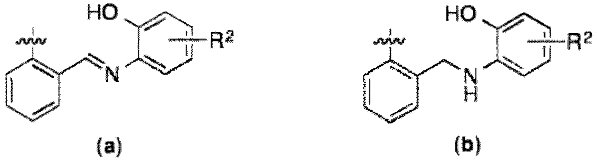
où, dans les formules (a) et (b),
R² est de l'H, de l'alkyle en C₁-C₆, de l'alcoxy en C₁-C₆, de l'halogène, du OH, du NH₂, du -CN, de l'aryle, du SH ou du -S-alkyle (C₁-C₆),
où par alkyle il faut entendre également alcényle, alcynyle, et cycloalkyle ou cycloalkylalkyle saturé ou partiellement insaturé.

3. Composé de formule (I) selon la revendication 2, dans lequel R¹ est placé en position méta par rapport au groupe NH du même cycle aryle.

4. Composé de formule (I) selon l'une quelconque des revendications 2 et 3, dans lequel R¹ est le groupe - CO-.

5. Composé de formule (I) selon l'une quelconque des revendications 2 à 4, dans lequel X est du phényle ou du phényle éventuellement substitué.

6. Composé de formule (I) selon l'une quelconque des revendications 1 à 5, dans lequel Pep comporte 3 à 50, 3 à 40, 3 à 30, 3 à 25, 3 à 22, 3 à 20, 3 à 17, 3 à 15, ou 3 à 10 acides aminés.

7. Composé de formule (I) selon l'une quelconque des revendications précédentes, dans lequel le peptide est choisi dans le groupe qui est constitué par
- Arg Pro Lys Pro Gln Gln Phe Phe Gly Leu Met (SEQ ID NO: 1) (neuropeptide substance P) et des fragments d'un tel peptide
- GNGRG (SEQ ID NO: 2)
- (GNGRG)₂KNK (SEQ ID NO: 3)
- formyl MLF (le prototype de formylpeptides qui se lient aux récepteurs des neutrophiles)
- (VGVAPG)₃ (SEQ ID NO: 4)
- (VPGVG) (SEQ ID NO: 5)
- RALVDTLKFVTQAEGAK (SEQ ID NO:6)
- RALVDTEFKVKQEAGAK (SEQ ID NO: 7)
- PLARITLPDFRLPEIAIP (SEQ ID NO: 8)
- GQQHHLGGAKAGDV (SEQ ID NO: 9)
- PLYKKIIKKLLES (SEQ ID NO: 10)
- LRALVDTLK (SEQ ID NO: 11)
- GGGLRALVDTLK (SEQ ID NO: 12)
- GGGLRALVDTLKFVTQAEGAK (SEQ ID NO: 13)
- GGGRALVDTLKALVDTL (SEQ ID NO: 14)
- GHRPLDKKREEAPSLRPAPPPISGGGYR (SEQ ID NO: 15)
- PSPSPIHPAHHKRDRRQ (SEQ ID NO: 16)
- GGGFD (SEQ ID NO: 17)
- YWDKTFT (SEQ ID NO: 18)
- SYNRGDSTC (SEQ ID NO: 19)
- GGGLRALVDTLK (SEQ ID NO: 20)
- GCGGGLRALVDTLK (SEQ ID NO: 21)
- GCYRALVDTLKFVTQAEGA (SEQ ID NO: 22)
- GC(VGVAPG)₃ (SEQ ID NO: 23)
- neuropeptide Y (36 aa)
- somastine (92 aa)
- cholécystokinine (jusqu'à 95 aa)
- pancréozymine (jusqu'à 95 aa)
- bombésine (14 aa)
- galanine (30 aa)
- bradykinine (9 aa)
- angiotensine II (8 aa)
- peptide relié au gène de la calcitonine (CGRP) (37 aa).

8. Composé de formule (I) selon l'une quelconque des revendications précédentes, dans lequel le groupe de liaison BG est lié au groupe alpha-amino du résidu d'acide aminé N-terminal de Pep ou à une chaîne latérale d'un résidu d'acide aminé se trouvant à l'intérieur de Pep.

9. Composé de formule (I) selon l'une quelconque des revendications précédentes, dans lequel le support T est une résine qui est appropriée en tant que support solide dans un procédé automatisé de synthèse de peptides en phase solide.

10. Utilisation d'un composé de formule (I) tel que défini dans les revendications 1 à 9, pour la préparation d'un réactif d'imagerie, le réactif comprenant un métal choisi parmi ^{99m}Tc, ¹⁸⁶Re, ¹⁸⁸Re, ⁹⁰Y, ⁸⁶Y, ¹⁷⁷Lu, ⁶⁸Ga, ⁶⁷Cu, ⁶⁴Cu, ⁶⁷Ga, ⁸⁹Zr, ¹⁵³Sm et ¹¹¹In.

11. Utilisation d'un composé de formule (I) tel que défini dans les revendications 1 à 9, pour la préparation d'un agent de radiothérapie, l'agent de radiothérapie comprenant un métal choisi parmi ^{99m}Tc, ¹⁸⁶Re, ¹⁸⁸Re, ⁹⁰Y, ⁸⁶Y, ¹⁷⁷Lu, ⁶⁸Ga, ⁶⁷Cu, ⁶⁴Cu, ⁶⁷Ga, ⁸⁹Zr, ¹⁵³Sm et ¹¹¹In.

12. Utilisation selon la revendication 10, dans laquelle le réactif d'imagerie est utilisé pour l'emploi dans un procédé d'imagerie, le procédé d'imagerie étant de préférence la tomographie par émission monophotonique (SPECT, Single Photon Emission Computed Tomography) ou la scintigraphie.

13. Procédé pour la préparation d'un composé de formule (I)
T-Pep-BG (I)
dans laquelle T, Pep et BG ont les significations indiquées dans les revendications 1 à 9, le procédé comprenant :
a) la synthèse d'un polypeptide Pep, Pep étant un polypeptide qui est lié par covalence au support T et présente une séquence d'acides aminés comportant 3 à 200 acides aminés,
pour l'obtention d'un composé de formule
T-Pep ;
b) la mise en réaction de T-Pep avec un précurseur BG' de BG, pour l'obtention d'un composé de formule (I)
T-Pep-BG (I)
dans laquelle BG est un groupe de liaison (non complexé) pour un métal M, le métal M étant choisi dans le groupe qui est constitué par ^{99m}Tc, ¹⁸⁶Re, ¹⁸⁸Re, ⁹⁰Y, ⁸⁶Y, ¹⁷⁷Lu, ⁶⁸Ga, ⁶⁷Cu, ⁶⁴Cu, ⁶⁷Ga, ⁸⁹Zr, ¹⁵³Sm et ¹¹¹In.

14. Procédé selon la revendication 13, qui comprend encore :
c) la mise en réaction du composé de formule (I) avec un réactif qui comprend un métal M, pour la formation d'un composé de formule (II)
T-Pep-BG-M(Y1)(Y2)(Y3) (II)
dans laquelle Y1, Y2 et Y3 représentent chacun indépendamment CO ou Cl, ou l'un parmi Y1, Y2 et Y3 est O et les deux autres représentent indépendamment l'un de l'autre CO ou Cl, et
M est choisi parmi ^{99m}Tc, ¹⁸⁶Re, ¹⁸⁸Re, ⁹⁰Y, ⁸⁶Y, ¹⁷⁷Lu, ⁶⁸Ga, ⁶⁷Cu, ⁶⁴Cu, ⁶⁷Ga, ⁸⁹Zr, ¹⁵³Sm et ¹¹¹In ; et
d) éventuellement la coupure de la liaison entre T et Pep, pour l'obtention d'un composé de formule (III)
H₂N-Pep-BG-M(Y1)(Y2) (Y3) (III)

15. Trousse pour la fabrication d'un produit radiopharmaceutique ou d'une composition de radiodiagnostic, dans lequel le produit radiopharmaceutique ou la composition de radiodiagnostic comprend un composé de formule (III) défini dans la revendication 14, la trousse comprenant un composé de formule (I) tel que défini dans les revendications 1 à 8 et un réactif qui comprend un métal M qui est choisi parmi ^{99m}Tc, ¹⁸⁶Re, ¹⁸⁸Re, ⁹⁰Y, ⁸⁶Y, ¹⁷⁷Lu, ⁶⁸Ga, ⁶⁷Cu, ⁶⁴Cu, ⁶⁷Ga, ⁸⁹Zr, ¹⁵³Sm et ¹¹¹In, pour la formation d'un composé de formule (III).
